# EUROPEAN PATENT APPLICATION

(11) **EP 3 817 002 A1**
(43) Date of publication of application: **05.05.2021**
(21) Application number: 19206146.3
(22) Date of filing: 30.10.2019
(51) Int. Cl.: G16H 10/00, G16H 10/20, G16H 50/70, G06F 21/62, H04W 12/02

(54) **SYSTEM FOR ANONYMIZING PATIENT DATA**

(71) Applicant: Gotthardt Healthgroup AG, 69117 Heidelberg (DE)
(72) Inventor: GOTTHARDT, Daniel, 69117 Heidelberg (DE); PETER, Sven, 69117 Heidelberg (DE)
(74) Representative: Richardt Patentanwälte PartG mbB

(57) **Abstract**

The invention relates to a computer system (100) for the anonymization of personal data. The computer system comprises a control computer system (128), a provisioning computer system (128, 262) and at least one user computer system (160). The control computer system comprises a control software (140) for providing anonymized personal data to an analysis software (139). The analysis software comprises a plurality of different analysis functions (132-138). The provisioning computer system comprises a plurality of anonymization protocols (121) each associated with one of said plurality of different analysis functions (132-138). Each of the anonymization protocols is configured to select and anonymize personal data in a manner adapted to the one analysis functions associated with said analysis protocol. The user computer system comprises a data store (102) in which personal data (104-110) is stored and an anonymization software (114).

The anonymization software is configured for:
- receiving at least one anonymization protocol (120);
for each of said at least one anonymization protocol:
- selecting and anonymizing a subset of the personal data in accordance with said anonymizing protocol; and
- transferring the anonymized subset and an identifier of the anonymization protocol to the control software.

The control software is configured for:
- receiving the at least one anonymized subset and the at least one identifier from said anonymizing software; and
- providing the subset and the identifier to the analysis software for performing those analysis functions to which the anonymization protocol identified by the identifier is associated, on the subset.

## Description

### TECHNICAL FIELD

The present disclosure concerns a method and system for the secure administration of personal data and in particular a method and system for the anonymization of personal data.

### BACKGROUND

In many countries, personal data enjoy special legal protection, i.e. their disclosure to third parties is not permitted or only permitted under certain conditions. Personal data is often highly sensitive. For example, the data collected in the healthcare sector, e.g. in doctors' surgeries and hospitals, but also in health insurance companies, is personal and particularly in need of protection. Similarly, the data collected by law firms, state authorities, companies and political associations from their clients, customers or members is personal and often highly sensitive. In order to protect them from unauthorized access, various techniques are used, e.g. encryption, anonymization and storage in particularly secure data stores and computer systems.

On the other hand, there is an increasing need to make the knowledge hidden in personal data usable for various purposes. With the emergence of new computer-based technologies for the efficient processing and analysis of large amounts of data (in particular in the field of "Big Data" and "Artificial Intelligence"), new possibilities have been created to extract knowledge from personal data which is of great importance and benefit both to the individual and to the general public. In the medical field, for example, there are numerous medical studies, e.g. with regard to the compatibility of a certain drug with other drugs or with regard to the influence of certain environmental or nutritional factors on a certain disease or health state in general. As a rule, both sides benefit if a patient takes part in such a medical survey: the survey gains in quality because the number of participants and thus the breadth of the database increases. In many survey, the patient benefits from the fact that he or she receives closer medical care and benefits from the findings of the survey even earlier than other patients who do not participate in a survey. Also in other areas (university studies on various aspects of human behavior, for example purchasing decisions, voting decisions, social relationships, etc.) it may be attractive for both sides to make personal data available to an analysis service.

The use of the large amount of personal data already available for analytical purposes, however, is prevented by the fact that the persons and/or organizations that collect and store personal data may not and/or do not wish to pass them on to third parties. Personal data is often stored in computer systems that do not allow the export or other automated transfer of data to third parties for security reasons. For example, computer systems of clinics and/or GP practices often comprise sensitive patient data that are protected by technical and/or organizational measures.

In practice, however, an on-site analysis within this particularly secure computer system is often also not an option:
On the one hand, the providers of special analysis programs are often small to medium-sized companies specializing in a niche market. They often do not enjoy the trust of the large software manufacturers such as Microsoft, SAP or Apple. The installation of third-party software in the context of a high-security computer system with sensitive personal data is seen by many administrators as critical and prevented for (not unfounded) fear of malware. In addition, the providers of complex analysis tools often do not provide the executables and rather offer the analysis as a service only, e.g. via the internet. And even if an analysis software is freely available and considered trustworthy, the installation of a separate analysis software for each individual analytical question, which might be relevant in the context of a certain type of personal data, would often entail an effort for the technical personnel that is too high for the operator of the secure infrastructure to store the sensitive personal data.

Various state-of-the-art methods for anonymizing personal data are known. The anonymization of personal data is intended to find a compromise between the often existing interest to make personal data available for analysis at least in an anonymized form and the need to protect the privacy of a person.

In the medical field, for example, the "NLM-Scrubber" program is available to remove personal information in medical free text documents.

Patent application WO2019097327A1 describes the anonymization of patient data. However, the use of anonymization programs in practice is often not possible or only possible to a limited extent due to the aforementioned problems: security precautions prevent or make it an extremely time-consuming process to install new software, for example for the purpose of anonymization. Even if, in individual cases, it is possible to install anonymization software in a security-critical area, for example in order to be able to carry out a certain analysis on the anonymized data, which appears to be particularly important, the usability of such an anonymization system is often only possible to a very limited extent in terms of both the subject matter and time, since the installed anonymization software can only be adapted with considerable effort while maintaining data security. However, the enormous dynamics in the field of data analysis mean that any anonymization concept developed for a specific purpose or analysis quickly proves to be outdated or unsuitable.

### SUMMARY

Current anonymization systems and the methods developed for anonymizing personal data often cannot be used due to security reasons or require a highly specific and time-consuming adaptation to a specific purpose. They are often characterized by complexity, inflexibility and poor extensibility.

The invention provides for an improved method, computer program product and system for anonymizing personal data as specified in the independent patent claims. Embodiments of the invention are given in the dependent claims. Embodiments of the present invention can be freely combined with each other if they are not mutually exclusive.

In one aspect, the invention concerns a computer system for anonymizing personal data. The computer system comprises a control computer system, a provisioning computer system and one or more user computer systems.

The control computer system includes control software for providing anonymized personal data to at least one analysis software. The at least one analysis software contains a multitude of different analysis functions for the analysis of personal data.

The provisioning computer system contains a multitude of anonymization protocols, each of which is assigned to one of the multitude of different analysis functions. The anonymization protocols are each configured to select and anonymize personal data in a manner adapted to the respective assigned analysis function.

The user computer system is connected to the control computer system and the provisioning computer system via a network. According to some embodiments, the control computer system and the provisioning computer system are different computer systems. According to other embodiments, the control computer system and the provisioning computer system are identical, meaning that both functionalities are implemented in a single computer system. The user computer system includes anonymization software and a data store. Personal data is stored in the data store in a non-anonymized and protected form.

The anonymization software is configured to receive at least one anonymization protocol of the variety of anonymization protocols from the provisioning computer system. The anonymization software is also configured to select and anonymize a sub-set of the personal data for each of the at least one anonymization protocols, whereby the selection and anonymization takes place according to the anonymization protocol, and to transfer the anonymized subset and an identifier of the anonymization protocol used for anonymization to the control software. For example, the selected sub-set can be a sub-set of the personal data of a particular person.

The control software is configured to receive the at least one anonymized subset and the at least one identifier from the anonymization software and to provide the at least one anonymized subset and the at least one received identifier to the analysis software. The provision is carried out in order to enable the analysis program to perform the analysis function to which the anonymization protocol identified by the identifier is assigned. The analysis function is performed on the provided subset of the personal data.

This can be advantageous since a system is provided which is highly flexible and expandable with respect to a multitude of different analytical questions and which is particularly suitable for providing personal data in anonymous form for a multitude of different analysis functions with different requirements with respect to data format and data content.

Many technical areas in which computer-based, complex analyses are performed are characterized by a high heterogeneity of both the existing personal data and the existing analysis algorithms and their requirements for data format and content. The forms of implementation of the invention also make this complexity manageable in the context of a high-security computer architecture, in that the anonymization software contains one or more anonymization protocols, each of which is assigned to a specific analysis function, and which are configured to both select and anonymize data in a manner specifically adapted to the analysis function. The connection of the aspect of the data selection with the aspect of the anonymization within a protocol and the assignment of these protocols to certain analysis functions makes it possible, flexibly depending upon the needs of the respective analysis to select aimed such data from the entire data stock, which are suitable for an analysis (particularly), and to anonymize these in such a way that sensitive data of a person, e.g. a patient, is protected, and is nevertheless guaranteed that the analysis can be accomplished.

In the field of medicine, for example, various medical practices and clinics use different software programs to enter and manage patient data. These differ with regard to the data formats used, the data fields queried, and much more. For example, different characteristics of a patient are relevant for a cardiologist than for a neurologist or orthopedist. An x-ray diagnostic department will usually store x-ray images as part of the patient file, whereas a geriatric clinic will capture a dementia-related patient image rather in the form of a natural language description of the patient's memory performance. An ear, nose and throat doctor will ask his patients different questions and record different data than an internist, pediatrician or oncologist. As a rule, different doctors use different programs to collect relevant patient data or at least different user interfaces. The database of a joint practice and even more the database of clinics and hospital associations can therefore contain personal, medical data of a large number of patients, which are extremely heterogeneous with regard to the recorded attributes.

The analysis functions available in this area are also extremely heterogeneous: there are programs that automatically calculate current and future diagnoses based on a patient file. There are image analysis programs that use digital images of histological tissue sections to automatically detect whether a tumor is present and, if so, what type of tumor it is, detect skin cancer on images of human skin, or detect breast cancer nodes in mammographic images. There are analysis programs that can detect statistical correlations of a variety of parameters, for example, an increased incidence of certain disease symptoms when taking a certain drug, a certain diet, a certain place of residence, a certain genetic marker, or the combined intake of two or more drugs. The breast cancer detection program does not require information about the patient's place of residence or images of the skin, but necessarily mammographic data. A statistical analysis program to be used to detect a possible correlation between lung cancer and whereabouts (inside or outside cities, near or far from a highway, etc.) based on demographic data, requires the address information of individuals, as well as information about whether lung cancer or other respiratory disease has been diagnosed, but does not require details such as x-rays or tissue section images.

The use of anonymization protocols, which select and anonymize personal data in a way that is required by a particular analysis function, thus makes it possible to provide personal data in a very flexible manner in a secure form for a variety of different analytical tasks.

In a further beneficial aspect, the processing of sensitive personal data (e.g. for anonymizing the personal data) takes only place locally on a computer system already comprising or having access to the sensitive personal data, e.g. the computer of a lawyer or the computer in the doctor's practice, and only anonymized data is transferred from this computer to another computer configured to perform one or more analyses.

The fact that the protocols provide for an analysis-specific selection and anonymization of the data can therefore be advantageous, since enormous flexibility is made possible with regard to a large number of current and future analysis functions.

Preferably, the protocols are configured in such a way that they selectively select and anonymize only those personal data that are absolutely necessary for the respective analysis function.

This can be advantageous, since a particularly high level of protection for sensitive data is achieved by the fact that a large part of the existing personal data is not selected from the outset and transferred to the control software. In addition, this can significantly reduce the response time of the system and the data traffic, because the less data selected, the less has to be processed in the course of anonymization and then transmitted via a network to the control software.

Embodiments of the invention therefore allow an advantageous compromise between data security on the one hand and flexible support for as many different analysis functions as possible on the other. The transfer of anonymized personal data for analysis purposes has several significant advantages for the general public: the availability of large data sets comprising a large number of different people is a prerequisite for a scientific, data-based analysis of important questions of fundamental importance, for example with regard to the influence of certain environmental pollutants on health, or the presence of side effects of a certain drug in long-term use.

However, individual patients may also benefit from participating in a survey in which their personal data is anonymously shared with the investigator. For example, participants in such studies regularly benefit from an even more accurate collection and description of relevant health-related data and/or from the opportunity to get in touch with a person who is particularly familiar with the respective medical question (survey leader). A growing number of complex medical questions can now be assessed more accurately by special analysis programs than by a physician. This applies to various forms of image analysis, but also to the intake of a variety of drugs, the possible interactions of which can often no longer be predicted even by a trained physician. Computer-based analysis functions can help people with many comorbidities, for example, to find an individually tailored and tolerable combination of drugs. The analysis functions can include analysis functions for predicting precision therapies for complex diseases and/or functions for identifying patients who meet the requirements to participate in a survey. The analysis functions can be provided and continuously updated by multiple vendors and/or a central healthcare provider, so that a physician can use these analysis functions to stay up to date despite the rapid increase in medical knowledge and available analytical software programs and features.

Depending on the model, the analysis functions include one or more image analysis functions, for example for cancer detection and/or classification (based on digital images of e.g. human skin, tissue sections, x-rays, etc.).

According to some embodiments, the protocols are non-executable data objects, e.g. ASCII files, in particular JSON or XML or YAML files. For example, the control software or a separate software installed on the control computer or the provisioning computer can comprise an editor with a GUI enabling a user to use a GUI to inspect and edit each of the protocols. This may have the advantage that any edits of a protocol are immediately effective without the need to recompile any program code.

According to some embodiments, the analysis functions include one or more contextual analysis functions, each of which is configured to jointly analyze a variety of data points of different types (for example, image data, text data, structured metada-ta such as location, age, gender, etc.) to identify context-relevant information.

According to embodiments of the invention, the analysis functions include one or more functions for identifying correlations between two or more data points, where a data point includes, for example, the intake of a particular drug, a particular diet, attributes of the patient (for example, age, place of residence, gender, eye color, height, weight, hair color, pre-existing conditions). This can be advantageous because these analytical functions can identify scientifically and socially relevant factors that have a positive or negative impact on health and thus contribute to generating new knowledge, promoting health and reducing medical costs.

According to embodiments, the control computer system also comprises the analysis software. However, it is also possible that the analysis software is instantiated on another computer system connected to the control computer system via a network. It is also possible that the variety of analysis functions can be performed on multiple analysis computer systems. For example, a clinic specializing in oncology could perform image-based analysis of images of tissue sections, while an environmental institute could perform demographic analysis of the correlation between various environmental toxins and health on its computers. Even in case the analysis functions are distributed to different computer systems and possibly also different organizations, the identifiers of the analytical functions and respective anonymization protocols are unique. In this case, the plurality of anonymization protocols are administered by the control computer system cross-system and cross-organization wise. The control software also contains information regarding the addresses and interfaces of the respective analysis computer systems and is configured to forward the anonymized subset of the personal data generated by a specific anonymization protocol to exactly the one of a plurality of analysis computer systems which comprises the analytical function to which this anonymization protocol is assigned.

The analysis software is configured to perform those analysis functions that are identified by the identifier provided by the control software.

According to some embodiments, the result of this analysis can, for example, only be displayed to the operator of the analysis computer (who, for example, may or may not agree with the operators of the control computer system). For example, the results of a correlation analysis regarding the side effects of a drug can be displayed to the survey leader, often the manufacturer of the drug. However, it is also possible for the results to be returned to the user's computer system, for example, to provide the results of the survey to a physician who has agreed to provide anonymous parts of the patient data he has collected for this survey. Although the user of the user computer system, e.g. a physician, cannot assign the result to a specific person due to anonymization, the result may nevertheless be very important for the physician. If, for example, the physician frequently prescribes the drug M1, he is obviously interested in the result of a large demographic study that is to determine the effects of another drug M2 on M1 and for which the physician has provided corresponding patient data in anonymous form after the patients concerned have given their consent. For example, the results can be output via a graphical user interface of the anonymization software. However, it is also possible for the user to be informed of the result in another way, for example by e-mail or via another software program that has been used, for example, to collect personal data. This other program may be, for example, a patient management program, a customer management program, or any other form of person management program. The other program may be operationally linked to the anonymization software.

According to embodiments, the control computer system serves as the provisioning computer system. This can be advantageous as the anonymized data does not need to be retransmitted over a network. This reduces data traffic and processing time and increases data security. For example, the control computer system with the integrated analysis software can be a server of a health service provider that integrates medical knowledge, current diagnostic procedures and therapy plans in analytical software in order to integrate the daily processes in clinics and practices of practicing physicians more efficiently, safely and profitably for the health of the patients. The interaction of the control software with the analysis software according to embodiments of the invention can facilitate the integration of clinical studies into the medical practice and thus both improve the database for the studies and allow a larger number of patients to benefit from the advantages of these studies.

According to embodiments, the computer system also includes personal data management software. The personal data management software is configured to interoperate with the anonymization software during the editing of personal data and/or during the input of new personal data by a user via a GUI to compare the data currently entered via the GUI and/or the input fields currently available in the GUI with the at least one anonymization protocol and to output a result of the comparison.

This can be advantageous, since at least one anonymization protocol of the anonymization software already influences the type of personal data collected during data entry and/or data maintenance. This can be very advantageous, especially if the system has been extended by a new analysis function from which, for example, the patients of a certain physician should benefit. It has been observed that many analysis functions have very specific, individual requirements with regard to the type and number of personal attributes. It is therefore possible that the existing personal data is not suitable for these analysis functions because the necessary information is missing. For example, the analysis can refer to the question of whether the side effects of a certain drug M1 are exacerbated when taking a rarely prescribed drug M2. Since the medicine M2 is rarely prescribed, the graphical user interface may not contain a separate field in which the intake of this medicine M2 can be marked with Yes or No. The doctor usually does not ask for this either. At most, it may happen sporadically that a patient has provided information on this. The anonymization protocol regarding the possible interaction of M1 and M2 could now automatically inform the physician when opening a patient file that he or she should ask the patient whether the patient takes M1 and M2. For example, a doctor's office that conducts a medical survey on the question of whether the combined intake of M1 and M2 has a negative effect on health can automatically ensure that the new or updated patient file contains clear information on whether the patient is taking or has taken M1 and/or M2 in the course of the usual patient appointments (e.g. for vaccinations, cancer check-ups or sick leave in the case of flu infections), when a new patient file is created or when an existing patient file is opened. Without significant additional effort for the physician and the patient, a data set is generated that contains all the data required for this medical survey. For example, the anonymization protocol for this medical survey can be configured to remove information about the patient's place of residence and store information about M1 and/or M2 intake in a structured manner. For example, both M1 and M2 could have a data field in the reduced, anonymized data set that contains a binary value (yes/no) related to the drug's ingestion, as well as additional fields for dose and duration of ingestion, if applicable.

It is therefore harmless, according to embodiments of the invention, that in many cases the input mask used by default does not contain input fields that refer to medical attributes that are not relevant in the vast majority of cases and outside the clinical trial. It is also not necessary (and not even possible in practice) for the physician to have an overview of all possible medical studies when creating a patient file, which he will make available anonymous patient data in the near or distant future in order to ensure that all necessary data is queried during data entry. Rather, the automatic interoperation of the personal data management software with the anonymization software during the editing of new or existing personal data on the basis of the anonymization protocol allows to compare the required data with the existing data or input fields and to inform the user of missing data. Medical studies often last from several months to several years, so that during this period, within the framework of the usual doctor consultations, the majority of the patients of a doctor who are eligible for a certain study will visit the practice anyway. On this occasion, the data sets of these patients can be supplemented with little effort for both doctor and patient. The patient can also agree to the use of his data on this occasion. For example, the physician can first ask the patient if he wants to participate in a particular medical survey and then send some of his data to the control computer system in anonymous form. Only if this is the case, the physician will activate the anonymization protocol for this patient that is part of this medical survey and add the information relevant to the study to the patient file. If the patient does not agree to the transfer of the data, the protocol is not activated for this patient's data and the data is not transferred to the control software.

According to embodiments, the personal data management software is installed on the same computer on which the anonymization software is installed. In other embodiments, the personal data management software is installed on a different computer than the one on which the anonymization software is installed, the other computer being located within the same security infrastructure as the application computer and providing for similar security measures with respect to the personal data entered as with respect to the already existing personal data.

Preferably, anonymization software and personal data management software are instantiated on the same computer system. This reduces the amount of data transmitted over a network and speeds up the process. For example, the anonymization software can be implemented as a so-called "plug-in" or "add-on" of the personal data management software. The "plug-in" or "add-on" is a software program which is implemented as an additional module of the personal data management software and can typically only be instantiated if the personal data management software has already been instantiated. Its appearance and functionality can be so closely matched to the appearance and functionality of the personal data management software that the user does not notice that the functionality has been added subsequently. It is also possible that anonymization software and personal data management software are two software modules of an application program which contains the two as software sub-modules.

According to some embodiments, the comparison of the data currently entered via the GUI with the anonymization protocol comprises:
- determining whether and which of at least one anonymization protocol(s) has been activated for the person whose personal data is currently being entered or ed ited;
- analyzing the one or more anonymization protocols activated for this person in order to determine the totality of all the attributes specified as a "necessary attribute" in all the anonymization protocols activated for this person, a "necessary attribute" being an attribute of a personal file required for the execution of the analysis function associated with the anonymization protocol;
- comparing the determined necessary attributes with the entered data;
- if the data entered does not contain at least one of the necessary attributes:
   - automatically outputting a warning message to the user; and/or
   - automatically modifying the GUI so that the modified GUI contains input fields for at least the missing "necessary attributes".

For example, the data values existing in all data fields and newly entered can be automatically searched for the presence of certain key terms defined in the anonymization protocol (for example, the names of the drugs M1 and/or M2 in the above example) when the patient file is opened or when the patient file is closed. If one or more of these key terms are recognized in the data values (e.g. a natural language text), the text is analyzed to determine the semantic statement contained therein, e.g. whether the patient explicitly denied or affirmed the use of M1 and, if so, to determine the dose and duration of use. If the automatic text analysis is able to extract the required information on these two drugs, the data required for the study is available and the user is informed of this or at least no warning message is issued. If the analysis reveals that the data is incomplete, a warning is issued or the user is informed in some other way that attributes are still required, and if so, which ones.

This embodiment can be advantageous, as it requires comparatively little interaction between anonymization software and personal data management software. Ultimately, the existing and, if applicable, currently supplemented data on a specific person are analyzed regardless of how the GUI for the maintenance of personal data is structured in detail. The user can also be notified independently of this GUI, e.g. through a popup window generated by the anonymization software, a loudspeaker message indicating the missing data, etc. The user can also be informed of the missing data by the GUI.

According to embodiments, the comparison of the input fields currently available in the GUI with the anonymization protocols comprises:
- determining whether and which of the anonymization protocols have been activated for the person whose personal data is currently being entered or edited;
- analyzing the one or more anonymization protocols activated for this person in order to determine the totality of all data fields specified as a "necessary data field" in all anonymization protocols activated for this person, where a "necessary data field" is a data field of a personal file that is used for storing an attribute required for the execution of the analysis function assigned to the anonymization protocol;
- comparing the determined necessary data fields with the data fields of the GUI;
- if the GUI does not contain at least one of the necessary data fields:
   - automatically issuing a warning message to the user; and/or
   - automatically modifying the GUI so that the modified GUI contains input fields for each of the required data fields.

This embodiment can be advantageous if a close interdependence and interaction of anonymization software and personal data management software is already in place, e.g. if the anonymization software is implemented as a plug-in or the anonymization and personal data management are implemented within the same software application. According to embodiments of the invention, at least one anonymization protocol, which is available in the anonymization software and which may have been activated for the currently created or modified personal data record, has an influence on the GUI used for the collection and maintenance of the personal data. The number and type of input fields contained in the GUI thus depends on the type and number of anonymization protocols contained in the anonymization software (and possibly specifically for a certain person whose data is currently being edited).

The GUI for data entry is adapted according to the protocols contained in the anonymization software and possibly activated for a specific person, thus ensuring that the user automatically collects all relevant data.

According to embodiments, one or more of the at least one anonymization protocols each contain a validity period. The validity period specifies a time of validity and usability of the respective protocol within the anonymization software. The anonymization software is configured to automatically collect the anonymized personal data in the form of a subset of the personal data in response to the end of the validity period in accordance with this protocol and to transfer it to the control software in collected form.

This can be advantageous, since the fact that the anonymized data is collected and transferred to the control software further reduces the possibility of deducing the identity of the respective persons. If, for example, a data record were transferred to the control software immediately after its creation or modification, and if the creation or modification would normally be connected with a visit of the person to the user of the user computer system, a connection between the anonymized data records and the respective person could be identified on the basis of the visit times of the person, who, for example, is known to another circle of employees of a doctor's practice or law firm, and the corresponding transfer times for the individual data records. The fact that the anonymized personal data is first collected and then transferred collectively prevents this. Preferably, the anonymization software is configured to collect personal data protocol-specifically and to transmit it to the control software only when a minimum number of persons have been anonymized with this protocol.

In addition, this feature can increase security by the fact that no further data records are collected and transmitted to the control software after the expiration of the protocol validity period. For example, the duration of the protocol can be linked to the duration of a medical survey or another study. This automatically prevents the collection and transmission of personal data that is no longer needed and processed by the recipient/the operator of the analysis software. This increases security, since otherwise, if the anonymization software contains a large number of protocols, it could happen that a user forgets to remove or deactivate the protocol at the end of a study for which the data was collected. By using a validity period, it can be ensured that the data transfer is automatically stopped without any active intervention by the user.

In some embodiments, the anonymization software includes a counter for one or more of the at least one anonymization protocols and updates it continuously. The one or more counters indicate in each case how many personal data records have already been anonymized with the anonymization protocol to which the counter is assigned. The anonymization software is configured to check whether one of the counters exceeds a predefined minimum value and, if the minimum value is exceeded, to automatically collect all the personal data already anonymized by the anonymization protocol assigned to this counter in the form of one of the subsets (also referred to as "batch") and transferring the subset of data anonymized by this anonymization protocol in the form of a batch of anonymized data records to the control software.

For example, a minimum number of e.g. 10 or more persons can be defined globally for all protocols. However, it is also possible that this minimum number is individually specified in each of the protocols or is individually specified in some of the protocols, whereby the protocol-specific minimum number then replaces the global minimum number.

According to embodiments, each protocol of the one or more anonymization protocols (i.e. the "at least one anonymization protocol") comprises:
- a specification of one or more "sensitive data fields", where a "sensitive data field" is a data field of a personal file whose original content is deleted or anonymized by the anonymization protocol in the course of anonymization; for example, fields for a person's first name and surname, telephone number, address and e-mail address are typically sensitive data fields. With some selected protocols, however, it is possible that at least parts of the address, such as the postal code and possibly also the street name, do not belong to the sensitive data fields, for example because the analysis functions assigned to the protocol necessarily require a location; and/or
- a specification of one or more "range data fields" and at least one respective associated value range, wherein a "range data field" is a data field of a personal file whose original content is replaced in the course of anonymization by the anonymization protocol by one of the value ranges defined in the anonymization protocol which comprises this data value; for example, it is generally not necessary to specify the exact age or the exact date of birth of a patient. However, age is often important in the medical context because it determines the probability of some diagnoses. By specifying different age group ranges, for example 0-5 years, 6-10 years, 11-15 years, 16-25 years, 26-35 years, 36-45 years, 46-55 years, 56-65 years, 66-75 years and over 76 years, or by replacing the actual age with a corresponding range value, the identity of a person can be hidden without completely renouncing the information content of the attribute; and/or
- a specification of one or more "necessary data fields", wherein a "necessary data field" is a data field of a personal file which is used for storing attributes that are necessary for the execution of the analysis function associated with the anonymization protocol; for example, an anonymization protocol which is to examine data for a study relating to a presumed increase of adverse side effects of the medicament M1 by another medicament M2, can indicate that the duration and/or amount of intake of M1 and M2 are "necessary data fields"; however, these data fields and respective attributes may be completely irrelevant for other analytical purposes and corresponding studies; and/or
- a specification of one or more "selection data fields" and at least one respective associated selection value, wherein a "selection data field" is a data field of a personal file whose content determines whether or not a data field of a personal file is extracted and anonymized in the course of anonymization; in particular, a "selection data field" is a data field of a personal file whose content is compared with the at least one selection value specified in the protocol during execution of the anonymization protocol, wherein a personal file is anonymized by the anonymization protocol only if the comparison reveals a sufficient similarity of the data content of the selection field with the at least one selection value; The anonymization software can be configured to analyze the personal data to determine whether the data in their selection data fields have sufficient similarity to one or more selection values defined in a protocol and to anonymize only personal data records with sufficient similarity and transmit them to the control software; For example, an anonymization protocol may be used to examine the influence of a particular diet before and during a woman's pregnancy on her child; in this case, an appropriate anonymization protocol could include the selection data field "gender" and the associated selection value "female" and the selection data field "pregnancy" and the associated selection value "within the last 5 years", so that the group of persons whose data is collected is limited to the group of female persons who are currently pregnant or have been pregnant within the last 5 years; and/or
- a mapping list comprising one or more synonyms mapped to a normalized term representing basically the same semantic content as the synonyms mapped to the normalized term, wherein all synonyms contained in a personal file are replaced with the normalized term to which the synonym is mapped in the protocol in the course of anonymization; The anonymization software can be configured to perform a protocol-based anonymization of a personal file such that the personal file is analyzed to identify the occurrence of one or more of the synonyms specified in the protocol and such that the synonyms are replaced by the normalized term to which the synonym is mapped for generating the anonymized data subset of this personal file; For example, the terms "male", "man" may both be mapped to the normalized term "man" and the terms "female" and "woman" may both be mapped to the normalized term "woman"; and/or
- a whitelist comprising a list of allowed data values which are to be maintained in the course of anonymization; when the anonymization software executes a protocol comprising a whitelist (that may be assigned to the whole personal file or individual fields), the anonymization software compares the terms in the whitelist with at least some data values of the personal data during execution of the anonymization protocol, and deletes selectively the data values which are not comprised in the whitelist; and/or
- a blacklist comprising a list of forbidden data values which are to be deleted or replaced in the course of anonymization; when the anonymization software executes a protocol comprising a blacklist (that may be assigned to the whole personal file or individual fields), the anonymization software compares the terms in the blacklist with at least some data values of the personal data during execution of the anonymization protocol, and deletes or replaces selectively the data values which are comprised in the blacklist; for example, the protocol may comprise multiple different blacklists, e.g. one or more field-specific blacklist; each blacklist defines a set of forbidden data values entered in a respective field. The anonymization software executing this anonymization protocol is configured to delete all entries entered in the field to which the blacklist is assigned that are part of the specified blacklist. The blacklist can be used e.g. to exclude very rare diagnoses that are irrelevant for statistics and that may be an obstacle in reliably anonymizing the data of a particular patient; all other diagnoses not listed in the blacklist may be included in the anonymized subset of the personal data in their original or amended form; and/or
- a time period indicating the granularity of an absolute-to-relative time conversion operation performed in the course of anonymization; the time period can be specified in the protocol on a per-field basis or globally for two or more different fields; when the anonymization software executes a protocol comprising a time period indicating the granularity, e.g. "year" or "month" or "day", the anonymization software automatically identifies all absolute times of a particular type of event, determines the time periods between these absolute times (i.e., the "relative time periods"), and stores the determined time periods in accordance with the indicated granularity; for example, the event type may be "doctor visits" or "surgeries" or "diagnoses"; each of these events may be stored in a personal file in association with a timestamp that allows computing the time periods between events of the same event type; these "relative" time periods do not allow reconstructing the original, absolute times which may be unique and characteristic for a particular person; nevertheless, the relative times represent a kind of "anonymized" time information that may give some useful information of the frequency of relevant events without revealing the identity of a person; and/or
- an identifier of the analysis function assigned to the anonymization protocol; and/or

According to embodiments, the anonymization software is configured to identify the one or more above-mentioned anonymization protocol elements (e.g. blacklists, whitelists, sensitive data fields, range data fields, time periods, mapping lists, identifiers, etc.) and perform the anonymization in accordance with these elements as described above.

According to embodiments, the personal data consists of a large number of personal files. The anonymization software is configured to:
- receiving a request for personal data from the control software, whereby the request contains an identifier of one of the anonymization protocols;
- performing said one anonymization protocol in response to receipt of said request, said one anonymization protocol comprising a specification of one or more "selection data fields" and at least one respective associated selection value, wherein performing said one anonymization protocol comprises comparing the content of said "selection data field" of all personal files with said at least one selection value, wherein said one anonymization protocol is configured to anonymize only those personal files for which said comparison results in sufficient similarity to said at least one selection value; and
- transmitting the anonymized personal files as the subset of the personal data to the control software, each together with an identifier of the one anonymization protocol.

The identifier can be implicitly implemented, e.g. in the form of a session ID of a user-related session between anonymization software and control software, where the session of the control software reveals the identity of the operator of the anonymization software and where the control software can use a registration database to identify a single anonymization protocol that was transmitted to the anonymization software after the user of the anonymization software had registered with the operator of the control software. Preferably, however, the identifier is explicit, i.e. a data value that is valid independently of a session and that is permanently assigned to an anonymization protocol from a large number of anonymization protocols.

According to some embodiments, the provisioning computer system is configured to receive a download request for at least one of the plurality of anonymization protocols; and in response to the receipt of the request, to transmit the at least one anonymization protocol via the network to the user computer system.

According to embodiments, the user computer system is configured to send the download request to the provisioning computer system.

For example, the anonymization software may be configured to send a download request regarding one or more anonymization protocols to the provisioning computer system at any time during the runtime of the anonymization software. The provisioning computer system may have a registry database in which it is stored for a large number of user computer systems or their operators which anonymization protocols are to be made available to them. After receiving the download request, the provisioning computer system checks whether the one or more anonymization protocols requested may be provided according to the registration database and, if permitted, makes the one or more anonymization protocols requested available to the requesting anonymization software via the network. This can be done by push or pull procedure.

According to embodiments, the computer system comprises a proxy computer system. The proxy computer system is connected via the network to the control computer system and to a plurality of user computer systems including the aforementioned at least one user computer system. Each user computer system comprises an instance of the anonymization software. Each of the plurality of user computer systems is connected to the control computer system only indirectly via the proxy computer system. The anonymized subsets and protocol identifiers are transferred from each of the anonymization software instances to the control software via the proxy computer.

According to embodiments, the proxy computer is configured to perform the transfer such that the identity of the one of the user computers having provided any one of the anonymized subsets and protocol identifiers is hidden from the control computer.

This may be advantageous, as it may ensure a higher degree of anonymity: for example, some doctors' practices and individual doctors have specialized heavily on a particular disease group or illness, so the IP address of the doctor's computer may be sufficient to reveal highly sensitive data of a patient that must not or should not be disclosed. Because the proxy computer does not forward to the control computer system any data that indicates the identity of the user computer, in particular IP addresses and other unique identifiers regarding the user computer or it's user, the control software cannot recognize from which user computer system it is currently receiving anonymized personal data. This may significantly increase the security of the transferred anonymous data.

In addition, or alternatively, the anonymization software instantiated on each of the user computer systems is configured to encrypt the anonymized subset of the personal data such that the control software but not the proxy computer can decrypt the transferred anonymized subset of the personal data.

For example, a private cryptographic key ("decryption key") can be stored in protected form in the control software. A copy of a public cryptographic key ("encryption key") is stored in each instance of the anonymization software installed on a user computer system. The encryption key and the decryption key together form an asymmetric cryptographic key pair. Each instance of the anonymization software is configured to encrypt the generated anonymized subsets of the personal data with the public encryption key before the data is sent to the control software (directly or via the proxy computer). Since the private decryption key is stored protected, i.e. inaccessible to the proxy computer or other unauthorized computer systems, and since the anonymized data is transmitted encrypted via the proxy to the control software, the proxy computer cannot read the transmitted data. This ensures the confidentiality of the anonymized data. The control software is configured to decrypt the received encrypted anonymized data with the private decryption key before the decrypted anonymized is forwarded to the analysis software.

A combination of the use of a proxy computer, which hides the identity of the user computer systems collecting or generating the data from the control computer system, with an encrypted transmission of the anonymized data can be particularly advantageous, since the origin of the data is concealed from the control software and thus an even higher degree of anonymization or data security is achieved. Encrypted transmission ensures that the proxy computer does not become a security risk because it knows the identity of the user computer systems from which a certain packet of anonymized data is received. Thanks to encryption, the proxy computer cannot access the contents of the data.

According to embodiments, the anonymization software is configured to automatically determine the degree of anonymization achieved by the execution of a particular anonymization protocol. Only if the anonymized data guarantees a predefined minimum degree of anonymity, the anonymization software transfers the anonymized data to the control software.

In addition, or alternatively, the control software is configured to automatically determine the degree of anonymization of the transferred anonymized subset received from the anonymization software. The control software is configured to provide the at least one anonymized subset and the at least one received identifier to the analysis software only in case the anonymized data guarantees a predefined minimum degree of anonymity.

These checks may have the advantage of protecting data that has not sufficiently been anonymized. For example, if a patient has a very rare disease or even has a combination of multiple rare features such as particular high age, a combination of one or more rare diseases and a combination of one or more rarely prescribed drugs, it may often be difficult or even impossible to reach an acceptable degree of anonymization of the data of this patient. In this case, performing one or more checks may ensure that sensitive patient data also of these patients are protected and may in addition allow automatically re-applying additional or more effective anonymization procedures on personal data having an insufficient degree of anonymization.

As a measure for the degree of anonymity, k-anonymity and/or I-diversity, for example, can be calculated and compared with a reference value indicating the required minimum degree of anonymization.

L-diversity is a form of group based anonymization that is used to preserve privacy in data sets by reducing the granularity of a data representation. The L-diversity approach is described e.g. in Aggarwal, Charu C.; Yu, Philip S. (2008). "A General Survey of Privacy-Preserving Data Mining Models and Algorithms", Privacy-Preserving Data Mining - Models and Algorithms. Springer. pp. 11-52. ISBN 978-0-387-70991-8. The reduction of granularity is a tradeoff that results in some loss of effectiveness of data management or mining algorithms in order to gain some privacy. The I-diversity model is an extension of the k-anonymity model which reduces the granularity of data representation using techniques including generalization and suppression such that any given record maps onto at least 1-1 other records in the data. The I-diversity model handles some of the weaknesses in the k-anonymity model where protected identities to the level of k-individuals is not equivalent to protecting the corresponding sensitive values that were generalized or suppressed, especially when the sensitive values within a group exhibit homogeneity.

K-anonymity is a property possessed by certain anonymized data. The concept of k-anonymity was first introduced by Latanya Sweeney and Pierangela Samarati as an attempt to solve the problem: "Given person-specific field-structured data, produce a release of the data with scientific guarantees that the individuals who are the subjects of the data cannot be re-identified while the data remain practically useful." (see e.g. Samarati, Pierangela; Sweeney, Latanya (1998), "Protecting privacy when disclosing information: k-anonymity and its enforcement through generalization and suppression", Harvard Data Privacy Lab., Retrieved April 12, 2017). A release of data is said to have the k-anonymity property if the information for each person contained in the release cannot be distinguished from at least k - 1 individuals whose information also appear in the release.

According to embodiments,, the k and l parameter are set to the following parameter values: k=10 and l=3-

According to a further embodiment, the anonymized data is transmitted in the form of "batches" from the anonymization software to the control software, whereby a batch is only transmitted if the anonymized data comprises at least a predefined minimum number of persons.

This may ensure that the transferred anonymized data cannot be linked to an individual person whose data might have been anonymized during an appointment with the operator of the anonymization software.

According to embodiments, the provisioning computer system comprises a private cryptographic signing key. Each of the plurality of anonymization protocols comprises a signature generated with the private cryptographic signing key. The anonymization software comprises a public signature verification key that forms an asymmetric cryptographic key pair with the private cryptographic signing key. The anonymization software is configured to verify the signature of each received protocol and for using any of the received anonymization protocols for selecting and anonymizing a subset of the personal data only in case the signature is valid.

For example, the signatures can be generated using ECDSA (Elliptic Curve Digital Signature Algorithm) or RSA.

This may ensure that no one can import fraudulent protocols into the anonymization software. This is because even if an attacker hacked the provisioning server and introduced a fraudulent protocol into the totality of protocols stored there, or if an attacker hacked the user computer and imported a fraudulent protocol into the anonymization software, such a fraudulent protocol could not do any harm: the signature check would show that this protocol has no valid signature and would therefore never be executed. These features may ensure that a fraudulent protocol does not transmit sensitive data to another target server.

In a further aspect, the invention relates to a computer-implemented method for anonymizing personal data. The method is performed by a control computer system and a provisioning computer system (which is in some embodiments identical to the control computer system). The control computer system comprises control software for providing anonymized personal data to at least one analysis software, said at least one analysis software comprising a plurality of different analysis functions for analyzing personal data. The provisioning computer system comprises a plurality of anonymization protocols each associated with one of said plurality of different analysis functions. The anonymization protocols are each configured to select and anonymize personal data in a manner adapted to said associated analysis function.

The method comprises:
- providing, by the provisioning computer system, at least one anonymization protocol of the plurality of anonymization protocols to an anonymization software of a user computer system connected to the control computer system and the provisioning computer system via a network;
For each of said at least one anonymization protocols provided:
- receiving, by the control software of the control computer system, an anonymized subset of personal data of one or more persons and an identifier of the one anonymization protocol used by the anonymization software for selecting and anonymizing the subset, whereby the selection and anonymization was performed in accordance with said one anonymization protocol; and
- providing, by the control software, the at least one anonymized subset and the at least one received identifier to the analysis software for performing the one of the analysis functions on the subset which is associated with the anonymization protocol identified by the identifier.

In a further aspect, the invention relates to a computer-implemented method for anonymizing personal data. The method is performed by at least one user computer system connected to a control computer system and a provisioning computer system via a network, whereby according to some embodiments, the control computer system and the provisioning computer system are identical. The at least one user computer system comprises a data store in which personal data is stored in a protected, non-anonymized form. The at least one user computer system further comprises anonymization software. The control computer system comprises control software for providing anonymized personal data to at least one analysis software comprising a plurality of different analysis functions for analyzing personal data. The provisioning computer system comprises a plurality of anonymization protocols each associated with one of said plurality of different analysis functions. The anonymization protocols are each configured to select and anonymize personal data in a manner adapted to said associated analysis function.

The method comprises:
- receiving, by the anonymization software, the at least one anonymization protocol of the plurality of anonymization protocols from the provisioning computer system;

For each of said at least one anonymization protocol:
- selecting and anonymizing, by the anonymization software, a subset of said personal data, said selecting and anonymizing being performed according to said at least one anonymizing protocol; and
- transmitting, by the anonymization software, the anonymized subset and an identifier of the anonymization protocol used for anonymization to the control software for enabling the control software to provide the at least one anonymized subset and the at least one received identifier to the analysis software for performing the one of the analysis functions on the subset which is associated with the anonymization protocol identified by the identifier.

In another aspect, the invention relates to a computer-implemented method for anonymizing personal data. The method is executed by a control computer system, a provisioning computer system and at least one (one or more) user computer systems.

The control computer system includes control software for providing anonymized personal data to the at least one analysis software, wherein the at least one analysis software includes a plurality of different analysis functions for the analysis of personal data.

The provisioning computer system includes a plurality of anonymization protocols each associated with one of the plurality of different analysis functions, wherein the anonymization protocols are each adapted to select and anonymize personal data in a manner adapted to the respective associated analysis function.

The at least one user computer system is connected to the control computer system and the provisioning computer system via a network. The at least one user computer system contains a data memory in which personal data is stored in a non-anonymized form and anonymization software.

The computer-implemented method comprises:
- receiving, by the anonymization software, at least one anonymization protocol of the multitude of anonymization protocols from the provisioning computer system;
- for each of the at least one anonymization protocol:
- selecting and anonymizing, by the anonymization software, a subset of the personal data, wherein the selection and anonymization is performed according to the anonymization protocol;
- transmitting, by the anonymization software, the anonymized subset and an identifier of the anonymization protocol used for anonymization to the control software;
- receiving, by the control software, the at least one anonymized subset and the at least one identifier from the anonymization software; and
- providing the at least one anonymized subset and the at least one received identifier by the control software to the analysis software for performing those of the analysis functions to which the anonymization protocol identified by the identifier is associated, on the subset.

According to embodiments, the method also includes the execution of the analysis function by the analysis software.

In another aspect, the invention concerns a computer program product comprising computer-implemented instructions which, when executed by one or more processors, cause the one or more processors to perform a method for anonymizing personal data as described herein for embodiments of the invention or to perform one or more steps of this method.

The expression **"personal data"**, also known as personal information or sensitive personal information is any information that allows alone or in combination with other personal data to identify a person and/or that reveals sensitive patient-related information such as address, health status, illnesses, day and place of birth, political views and the like. Hence, any information that can be used to distinguish or trace an individual's identity, such as name, social security number, date and place of birth, mother's maiden name, or biometric records; and any other information that is linked or linkable to an individual, such as medical, educational, financial, and employment information, is "personal data".

The expression **"securely stored"** as used herein means that the securely stored data is secured from unauthorized access by one or more technical security measures, e.g. encryption, storing the data in a specially protected data center to which only a few reliable employees have access, etc.

The expression **"computer system"** as used herein is a machine or a set of machines that can be instructed to carry out sequences of arithmetic or logical operations automatically via computer programming. Modern computers have the ability to follow generalized sets of operations, called "programs", "software programs" or "software applications". These programs enable computers to perform a wide range of tasks. According some embodiments, a computer system includes hardware (in particularly, one or more CPUs and memory), an operating system (main software), and additional software programs and/or peripheral equipment. The computer system can also be a group of computers that are connected and work together, in particular a computer network or computer cluster, e.g. a cloud computer system. Hence, a "computer system" as used herein can refer to a monolithic, standard computer system, e.g. a single server computer, or a network of computers, e.g. a clout computer system. In other words, one or more computerized devices, computer systems, controllers or processors can be programmed and/or configured to operate as explained herein to carry out different embodiments of the invention.

A **"proxy computer**" as used herein is a dedicated computer system that serves as an intermediary between a data sending device, such as a user computer, and a data receiving device, e.g. a control computer receiving anonymized data from the user computer.

The expression **"field"** as used herein is a place where data of a particular category, e.g. a semantic or syntactic category, is to be stored or entered. For example, a data field of a GUI is a field where a value of a particular attribute, e.g. the name or address of a patient is to be entered. A data field in a database refers to a data structure region, e.g. a column of a database table, that is configured and used to receive and store data of a particular category that is assigned to this field.

The expression that an "anonymization protocol has been activated for a person" as used herein means that the anonymization protocol is stored in association with data, e.g. a flag or a property value or record in a configuration file or person file that indicates whether or not a particular anonymization protocol is allowed to be used for anonymizing personal data of this person and to provide the anonymized data to the control computer system. The activation of an anonymization protocol for a specific person does not automatically imply that data of this person is actually anonymized and sent to the control computer, because the person may, for example, not fulfill filter criteria that are defined in the protocol. However, if a protocol is not activated for this person, this always means that the data of this person is not anonymized with this protocol and transferred to the control computer.

A **"plug-in"** or "add-on" or "add-on" as used herein is a software component that adds a specific feature to an existing computer program. When a program supports plug-ins, it enables customization. Two plug-in examples are the Adobe Flash Player for playing videos and a Java virtual machine for running applets.

The embodiments and examples described herein are to be understood as illustrative examples of the invention. Further embodiments of the invention are envisaged. Although the invention has been described by way of example to a specific combination and distribution of software programs and computer systems, it is to be understood that any feature described in relation to any one embodiment may be used alone, or in combination with other features described, and may also be used in combination with one or more features of any other of the embodiments, or any combination of any other of the embodiments as long as these features are not mutually exclusive.

Accordingly, some embodiments of the present application are directed to a computer program product. Other embodiments of the present application include a corresponding computer-implemented method and software programs to perform any of the method embodiment steps and operations summarized above and disclosed in detail below.

Any software program described herein can be implemented as a single software application or as a distributed multi-module software application. The software program or programs described herein may be carried by one or more carriers. A carrier may be a signal, a communications channel, a non-transitory medium, or a computer readable medium amongst other examples. A computer readable medium may be: a tape; a disc for example a CD or DVD; a hard disc; an electronic memory; or any other suitable data storage medium. The electronic memory may be a ROM, a RAM, Flash memory or any other suitable electronic memory device whether volatile or non-volatile.

Each of the different features, techniques, configurations, etc. discussed herein can be executed independently or in combination and via a single software process on in a combination of processes, such as in client/server configuration.

It is to be understood that the computer system and/or the computer-implemented method embodiments described herein can be implemented strictly as a software program or application, as software and hardware, or as hardware alone such as within a processor, or within an operating system or a within a software application.

The operations of the flow diagrams are described with references to the systems/apparatus shown in the block diagrams. However, it should be understood that the operations of the flow diagrams could be performed by embodiments of systems and apparatus other than those discussed with reference to the block diagrams, and embodiments discussed with reference to the systems/apparatus could perform operations different than those discussed with reference to the flow diagrams.

In view of the wide variety of permutations to the embodiments described herein, this detailed description is intended to be illustrative only, and should not be taken as limiting the scope of the invention. What is claimed as the invention, therefore, is all such modifications as may come within the scope of the following claims and equivalents thereto. Therefore, the specification and drawings are to be regarded in an illustrative rather than a restrictive sense.

### BRIEF DESCRIPTION OF THE DRAWINGS

In the following, only exemplary forms of the invention are explained in more detail, whereby reference is made to the drawings in which they are contained. They show:
- Figure 1: a block diagram of an embodiment of an inventive computer system having a user computer system and a control computer system that also serves as a provisioning computer system;
- Figure 2: a block diagram of another computer system according to the invention with three user computer systems, one control computer system and one deployment computer system;
- Figure 3: a user computer system with a personal data management program and an anonymization plugin;
- Figure 4: a flowchart of a method for providing and using an anonymization protocol according to an embodiment of the invention;
- Figure 5: a flowchart of a method for collecting and anonymizing personal data in the course of opening a personal file;
- Figure 6: a flowchart of a method for providing and using an anonymization protocol, and
- Figure 7: a block diagram of a distributed system comprising multiple user computer system, a control computer system and a proxy.

### DETAILED DESCRIPTION

The following exemplary embodiments all refer to the medical field. However, the invention may also be used in other areas in which personal data is collected, stored and, under certain conditions, made available to third parties for external analysis. This applies in particular to the administration of clients, customers and members of an organization. When talking about "patients" here, "persons" are implicitly included and meant as well.

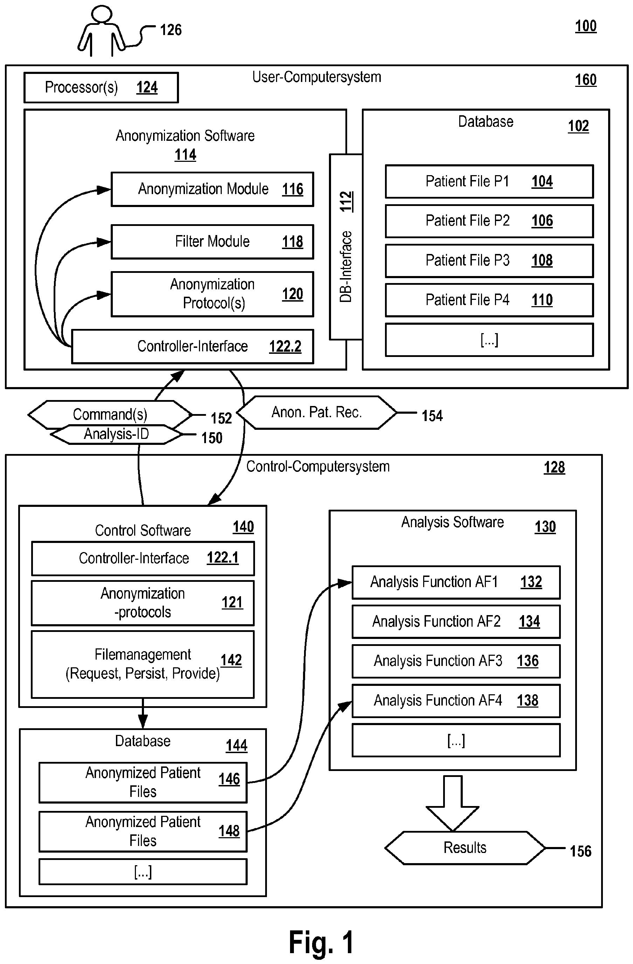

**Figure 1** shows a block diagram of an embodiment of an inventive computer system 100 with a user computer system 160 and a control computer system 128 that also serves as a deployment computer system.

For example, the application computer system may be a physician's computer, such as a single practice, a group practice, or a clinic or medical research facility. The computer system may include one or more processors and may be implemented as a notebook, smartphone, tablet computer system, terminal, server computer system, or distributed cloud computer system.

The user computer system contains a data storage 102, on which a large number of patient files 104-110 are stored in non-anonymous but protected form. The data storage can be any data storage, for example a file, or a set of files, or a file directory, or a database. Preferably it is a database, especially a relational database such as MySQL or PostgreSQL. For example, the data store can contain 102 personal data from a large number of people (in this case, patients). In addition, the user computer system 160 contains an anonymization software 114 which can access the personal data of the data memory 102 at least readably via an interface 112.

The anonymization software contains a multitude of functionalities. On the one hand, it contains an interface 122.2 to a provisioning computer system via which it can receive one or more anonymization protocols 120 via a network. Typically, the received anonymization protocols 120 represent only a small selection of the anonymization protocols 121 contained in the provisioning computer system 128. For example, the one or more anonymization protocols can be requested via interface 122.2 at any time during the runtime of the anonymization software and received via the network.

Each of the anonymization protocols 121,120 is clearly assigned to one analysis function 130 out of a multitude of analysis functions. This means that the anonymization protocol of a particular analysis function determines which personal data records are to be selected for a particular analysis and how this data is to be anonymized. The type of data selected and the type of anonymization is specific to the associated analysis functions, i.e., data collected and processed by another, unallocated protocol may not be processed or may not be processed correctly by an analysis function.

For example, each protocol can have a unique ID, a version ("revision") corresponding to a particular validity period, a start date and end date as indicated in the JSON example given below:

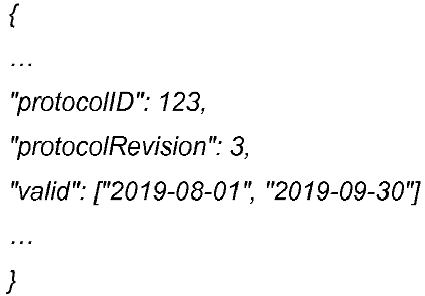

According to some embodiments, each protocol comprises one or more filter criteria that can be specified as filter rules. A filter rule is a function that specifies which attributes of a person file should be processed and how, and which specifies how to decide based on the result of this analysis if a person and his or her personal data is relevant for the analytical function and task to which this protocol is assigned. A pseudocode example for a filter rule is given below (the original JSON code would be less comprehensible):
*PatientFile.DiagnosisRecord containsHistoricalOrCurrent(24 months, [K75.8;K75.9;K76.0]) and Patient.Age < 85)*

Each filter rule may comprise an arbitrarily complex combination of Boolean operators.

According to some embodiments, one or more of the protocols respectively comprise a specification of a set of "quasi-identifiers".

A "quasi identifier" as used herein is an attribute of a person (or field name of a person file) which alone or in combination with other quasi identifiers bears the risk of making a person identifiable. For example, a diagnosis and/or medication of a patient can be a quasi identifier.

According to embodiments, to ensure a sufficient degree of anonymization, all quasi identifiers of a person together must be k-anonymous, otherwise in combination they can identify individual persons. For example, k can be 55 meaning that in a set of anonymized person data comprising e.g. the data of 10.000 persons, each person-specific combination of quasi-identifiers must be observed in at least 55 persons.

According to embodiments, the anonymization software is configured to automatically and dynamically repeat the anonymization procedure based on modified anonymization parameters, e.g. based on extended value ranges: in case the degree of anonymization obtained by replacing individual data values with respective data ranges is not sufficient, the replacing is repeated using larger value ranges, thereby increasing the number of persons in a person data set to which the attribute value ranges assigned to an individual anonymized person can be mapped. Examples for the dynamic computation of anonymization parameters can be found in the literature, e.g. in Aggarwal, Gagan, et al. "Approximation algorithms for k-anonymity. Journal of Privacy Technology (JOPT) (2005).

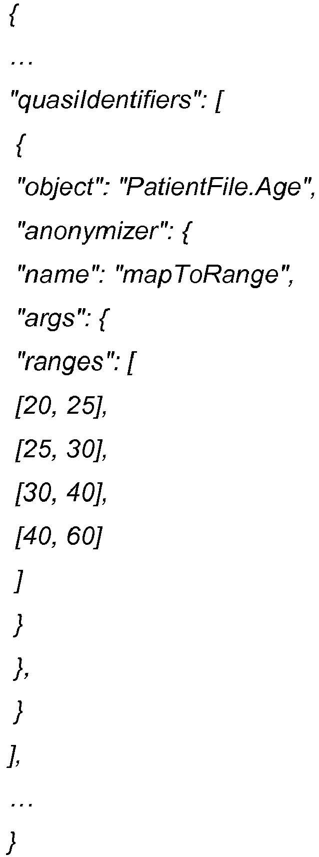

According to some embodiments, one or more of the protocols respectively comprise a specification of a set of "sensitive data" elements (i.e., "sensitive attributes" of a person comprising sensitive personal data which - in contrast to a "quasi identifier" - do neither alone nor in combination with other "sensitive data" elements make a person identifiable. However, these attributes may allow drawing conclusions on a group of persons. According to embodiments, the protocol can require an indication of a numerical number for the parameter "L", wherein "L" means -I-divers. All "sensitive data" elements of an anonymized patient record need to be I-divers to prevent drawing conclusions from the anonymized data records about an entire group of users.

A "sensitive attribute" or "sensitive data element" is an attribute of a person whose value for any particular individual must be kept secret.

A set of non-sensitive attributes can be or can acta s a "quasi-identifier" if these attributes can be used to uniquely identify at least one individual in the data set.

For example, let S denote the set of all sensitive attributes. An example of a sensitive attribute can be "medical condition". The association between individuals and "medical condition" hence needs to be kept secret and the anonymization process needs to ensure that the anonymized data does not allow linking a medical condition to an individual person or vice versa. Thus the sensitive data "medical condition=cancer" must not be disclosed in association with a particular patient but it may be permissible to disclose the information that cancer patients exist in a particular hospital.

A set of nonsensitive attributes of a table is called a quasi-identifier if these attributes can be linked with external data to uniquely identify at least one individual in the general population . One example of a quasi-identifier is a primary key, like social security number. Another example is the set {gender, age, zip code} in a data set comprising only a small number of persons per zip code. A zip code per se does not disclose sensitive data of a person, but in combination with other attributes it may reveal the identity of a person, thereby also disclosing the medical condition of this person.

For example, a data set to be anonymized may consist of all people in a small village. The data set comprises only 10 different 54 year old men who all suffer from disease X and it is known that there are only 10 54 year old men in this village. In this case, it is immediately known that "Max Mustermann" suffers from disease X as soon as I know that he is 54 years old. To prevent this, I-diversity is computed: If I=2 the group (54, man) should have at least two different entries for "has disease X". Embodiments of the invention use anonymization protocols configured to create anonymized data sets comprising as few "sensitive fields" as possible to handle data as sparingly as possible (e.g. to read only "Patient has diagnosis X" instead of a complete list of all diagnoses of a patient).

Accordingly, in order to ensure I-anonymity, only a subset of diagnoses from all existing diagnoses of a patient will be extracted and included in the anonymized patient record. For example, the subset of diagnoses can be the ones of the diagnoses of a patient mentioned on a whitelist specified in the protocol and/or can be the diagnoses observed within the last 12 months.

A protocol code sample for the sensitive data in JSON format is presented below:

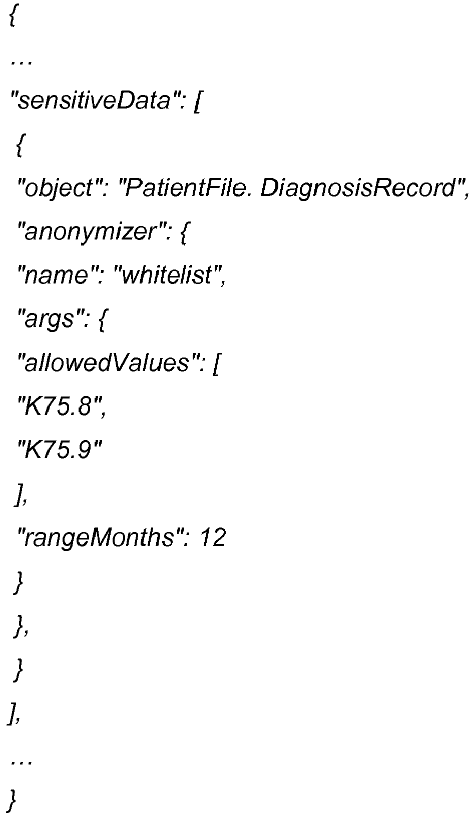

The received anonymization protocols 120 can be read by the anonymization software in order to select a certain subset of the existing patient files that are to be evaluated with regard to a certain analysis function. The anonymization software can, for example, use the anonymization module 116 to read in a certain anonymization protocol 120 and transfer it to a filter module 118, which uses the information in the protocol to select a subset of the patient files 104-110 that are suitable for the analysis functions assigned to the protocol. This selection of patient files is transferred from the filter module 118 to the anonymization module 116, which anonymizes the patient files of this selection according to the specific anonymization protocol. In the course of anonymization, data values in sensitive data fields in particular are completely removed, data values in range data fields are replaced by range information, necessary data fields specified in the protocol are checked to see whether the required information is available, and the anonymized patient data thus obtained is stored locally in a structured form that the control software 140 can process.

According to embodiments, the filter criteria are protocol-specific and are comprised in the protocols. In some examples, the filter criteria are automatically evaluated against the personal data when a person file is opened in a personal data management program 300 that is interoperable with the anonymization software. This may usually happen when a person (e.g. a patient) visits the operator of the anonymization software (e.g. a physician). If the patient is suitable for a protocol, the patient and the physician respectively have the possibility to object to the anonymization of this data. This objection will be saved and the data of this person will not be processed further by the anonymization software. Otherwise, a sub-set of the personal data selected in accordance with this protocol is read out, processed and stored anonymously in a local database by the anonymization software.

Preferably, a large amount of patient data is anonymized and stored locally as long as the validity period of the protocol 120 used has not expired. The expiry of the validity period of a protocol can be interpreted by the anonymization software 114 as a trigger signal to send all anonymized patient files having been generated by this protocol and having been stored locally in the form of a batch of anonymized patient records to the control software 140.

In addition or alternatively, it is also possible that the control software 140 triggers the transmission of the collected anonymized data records. This can be done, for example, by transmitting a command 152 from the control software to the anonymization software 114, whereby the command contains an identifier 150 of the analysis functions 132-138 to be performed and/or an identifier of the anonymization protocol assigned to these analysis functions. In response to the receipt of command 152, the anonymization software identifies an anonymization protocol which is assigned to identifier 150 directly or indirectly via the identifier of the analysis functions, executes the identified anonymization protocol(s) and provides a protocol-specific, anonymized subset of the patient data.

For example, the filter module 118 and anonymization module 116 can be used for selectively anonymizing and providing those patient data records which match some filter criteria (selection values) specified in the identified anonymization protocols. This subset 154 is returned to control software 140 in response to command 152.

According to embodiments, one or more of the protocols comprised in the anonymization software respectively comprise a specification of a data structure, e.g. of a database table, to be used for storing the anonymized data generated in accordance with this protocol. The data structure can be created dynamically by the anonymization software when or before performing the selection and anonymization based on the protocol. For example, the data structure can be created in a local database. Then, the anonymized subset of the sensitive data of one or more persons generated in accordance with this protocol is stored in this data structure in the local database.

If there is either enough data available (e.g. if more than a predefined minimum number of persons are represented in the anonymized data generated in accordance with a particular anonymization protocol) or if the defined validity of one of the anonymization protocols has expired, the data is transferred to the control software via the network. For example, the data can be transferred via a REST API in JSON format. Before transmission, the anonymizing software optionally checks whether the quasi-identifiers contained in the anonymized subset of the data that is to be transferred are k-anonymous and/or whether the "sensitive data" of this subset is I-divers. If this is not the case, the anonymization software transmits only an error message to the control software.

The control software 140 may include modules and functions 142 for managing the anonymized patient data received from one or more user computer systems, for storing this anonymized patient data 146, 148 in a database 144, and for providing the anonymized patient data specifically to selected analysis functions 132-138. The anonymized patient data is provided to selected analysis functions in such a way that an anonymized patient data subset 146,148 received by the anonymization software 114 is only provided to the analysis functions that are assigned to the anonymization protocol used to create the subset. For example, the control computer system may have a corresponding allocation table or allocation file that assigns a corresponding anonymization protocol to each of the analysis functions. The allocation table or allocation file may also contain address data from multiple analysis computer systems, if the variety of analysis functions 132-138 are distributed among multiple analysis computer systems. In this case, the control software selectively provides the subset received for a particular analysis function (it may also be multiple subset provided for an analysis function by a variety of user computer systems) to the address of the analysis computer system containing that analysis function.

According to some embodiments, the anonymized patient data subsets are transferred from the control software to the individual analysis functions of one or more analysis software programs 130 by means of push procedures. According to other embodiments, the anonymized patient data subsets are transferred from the control software to the individual analysis functions of one or more analysis software programs 130 by means of pull procedures.

After receiving one or more anonymous subsets of patient data, the analysis software 130 executes the corresponding analysis functions on this subset. The analysis functions can be performed in response to receipt of the subset, or after a sufficiently large data set has been received from one or more user computer systems for a particular analysis function. The result 156 returned by the analysis functions is output. The output is made to at least one user of the analysis computer system (which is identical to the control computer system here), for example via a screen, printer, or other user interface. These users may be, for example, the leader of a medical survey, researchers who have developed a particular complex statistical analysis, or anyone else who is in charge of implementing and/or performing an analysis.

In some cases, even if only for certain analysis functions, the result is also returned to the control software and issued to a user of the control computer system. The user of the control computer system may also be a leader of a medical survey, a person who has developed a particular analysis or integrated its use into the control software, or another person in charge of implementing and/or performing or integrating an analysis into the control software.

In some cases, in particular for analysis functions that evaluate a large amount of anonymized personal data within the framework of a scientific study, e.g. a medical survey, the result is also returned by the control software to the anonymization software 114, which has provided at least part of the anonymized personal data on the basis of which the results were obtained. Due to anonymization, the result cannot be assigned to an individual patient, but the user can still benefit from receiving the result, for example by being informed that a certain proportion of his patients have a particularly high or low chance of responding to a certain therapy and/or have a particularly high or low risk with regard to a certain diagnosis due to a specific diet, for example, or due to other characteristics that a doctor may observe in a patient.

According some embodiments, all communication between the control computer and each of the user computers is performed via an SSL/TLS connection. Preferably, the anonymization software and/or the person management software requires a user, e.g. a healthcare professional, to authenticate at the anonymization software and/or at the person management software (e.g. by providing a password, biometric data or other form of user credential).

The anonymization software can be configured to regularly synchronize its protocols with the protocols stored in the provisioning computer system and/or the control computer system to ensure the analysis software always comprises the latest version of the protocols already comprised in the anonymization software. According to some implementation variants, the synchronization comprises repeatedly (e.g. once a day) sending a request from the anonymization software to the control software via REST API to get a list of the most current version numbers of all currently active, locally available protocols. The synchronization can comprise receiving, by the anonymization software, a list of protocol identifiers from a remote computer (the provisioning computer system or the control computer system) indicating a number of protocols or protocol versions having been deleted on the remote computer. If an identifier of one of the anonymization protocols stored locally in the anonymization software is comprised in the list, the anonymization protocol automatically deletes this protocol and all locally stored anonymized data generated in accordance with this protocol. In case a newer version of one of the deleted protocols is available, the anonymization software automatically downloads this new version and verifies the signature of the downloaded protocol before the protocol is stored locally. For example, the anonymization software can comprise a public signature verification key that corresponds to a public root key of the organization that operates the control computer system and that typically also provides the anonymization protocols. The signature verification comprises checking a chain of signatures belonging to the Public Key Infrastructure of this organization, similar to e.g. a SSL/TLS PKI. If the signature is invalid or cannot be assigned to the root key, the protocol is not imported into the anonymization software and discarded. Otherwise, the new and verified protocol is used for evaluating and anonymizing personal data.

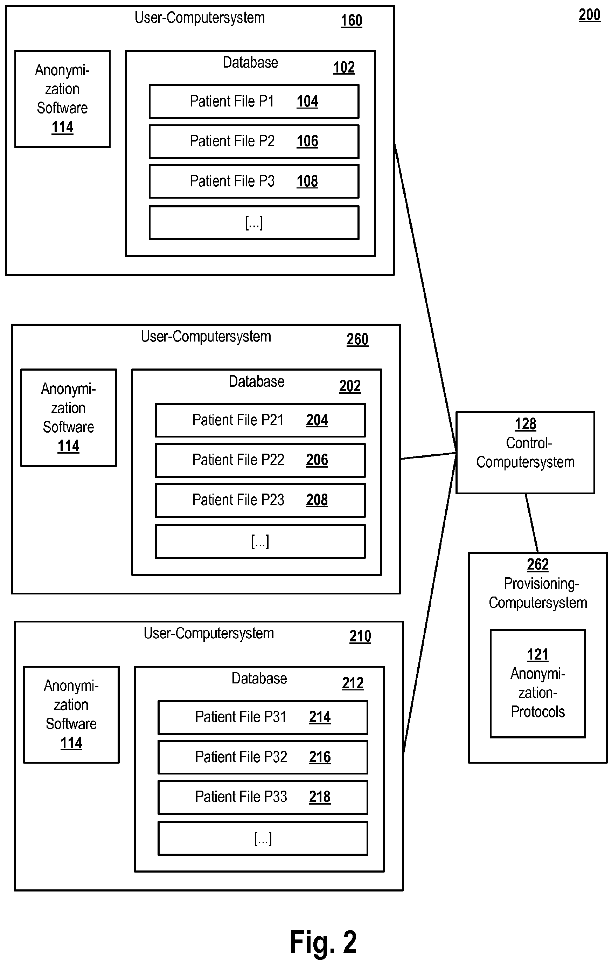

**Figure 2** shows a block diagram of another computer system 200 according to the invention with three user computer systems 160, 120, 260, a control computer system 128 and a provisioning computer system 262. It is a distributed computer system whose components are operatively connected to each other via a network, e.g. the Internet. Each of the computer systems 160, 120, 260, 128 and 262 can also be implemented as a monolithic or distributed computer system, e.g. as a computer network and/or as a cloud computer architecture. The user computer systems 160, 120, 260 each contain an instance of the anonymization software 114, which can exchange data with the control computer system via an interface, as described, for example, with regard to the embodiment shown in Figure 1. Each of the user computer systems contains a data store 102, 202, 210, e.g. a relational database in which personal data records are stored. Typically, the personal data records 104-108, 204-208, 214-218 of the different computer systems 160, 120, 260 originate from different persons and/or contain at least different contents. For example, user computer system 160 can be a computer of a general medical practitioner in Cologne, user computer system 260 can be a computer of a group practice in Berlin and user computer system 210 can be a computer in an oncology department of a hospital. Typically, the patient files therefore originate from different patients and/or differ at least with regard to parts of the contents of the patient files.

If, for example, the users of the user computer systems wish to participate in a study, e.g. a specific medical survey concerning the interaction of two drugs M1, M2, the users can obtain a corresponding anonymization protocol from the provisioning computer system, e.g. via a download link activated after conclusion of the contract, and import the protocol into the respective instance of the anonymization software 114.

According to some embodiments, the physician obtains the consent for the transfer of anonymous patient data from the respective patient when opening or creating a patient file. For example, creating or editing a patient file can automatically activate the protocol for this patient at least partly before the patient was asked to agree to the anonymization and forwarding of his or her data. This may have the advantage that the select value specified in the protocol can be evaluated and compared with the data content of the respective select field of the patient record before the user is asked for consent. For example, if the patient does not match the select value and does not "fit" in the survey, embodiments of the invention do not ask the patient for his or her consent to provide his or her data in anonymized form.

Often, an analysis function only refers to a certain group of people, e.g. people of a certain sex, age group, people with a certain pre-existing condition or long-term medication, etc. The analysis function is often used to determine whether the patient is a suitable candidate for the survey or the analysis function. In this case, the patient is only asked by the physician to agree to the data transfer if the patient belongs to the said group of persons.

If the patient does not agree to the anonymization and forwarding of his or her data to the control software/analysis software, the protocol will not anonymize this person's personal data and transmit it to the control software. If the partial execution of the protocol shows that the patient belongs to the group of people whose data can be used for the analysis function, the anonymization software instructs the physician to request all attributes relevant to this survey and specified in the protocol from the patient, e.g. by automatically modifying the fields of a GUI and/or outputting a visual, acoustic or other signal. After closing the patient file, the data of the patient that are relevant for the analysis function according to the protocol are selected and first stored anonymously locally in the respective user computer systems. In this way, each of the multiple instances of the anonymization software collects patient data and stores it locally until, for example, a minimum number of data sets has been collected and/or the validity period of the protocol has ended. Once one of these termination criteria has been met, the collected anonymized patient data is sent asynchronously from the individual instances of the anonymization software to the control software.

According to embodiments, the control software is configured to receive from a plurality of user computer systems 210, 260, 160 a set ("subset") of anonymized patient records obtained by executing a particular anonymization protocol, and to merge those records on a protocol-specific basis and provide them as a whole to the analysis function associated with that anonymization protocol.

In some versions, several 1000 or even several 10,000 application computer systems can be operatively connected to the control software and transmit anonymized patient data together with an identifier of the anonymization protocol used for anonymization to the control software. One or more different anonymization protocols can be installed and active in each of the user computer systems. The administration of the anonymized data of the individual user computer systems and the protocol-specific collection and combination of the anonymized patient data of several user computer systems can therefore be quite complex and require a sufficiently powerful computer architecture.

The type and number of anonymization protocols provided by the deployment computer system may change over time and must be synchronized with the type and number of analysis functions supported by the analysis software.

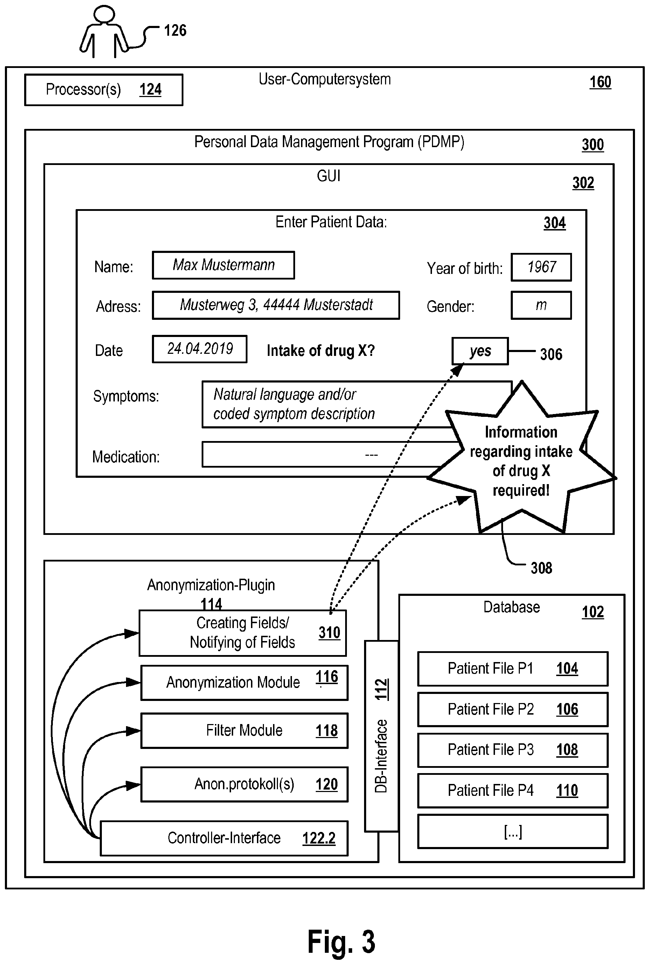

**Figure 3** shows a user computer system 160 with a personal data management program 300, e.g. a patient data management program, and anonymization software 114 designed as a plugin for this patient data management program. The patient data management program may include a standard input mask (graphical user interface - "GUI") that includes multiple input fields for personal attributes such as first and last name, address, gender and/or birthday, long-term medication, and current symptoms. The question of whether the patient is taking a particular medicine X is too specific to require a separate field in the standard input mask. Accordingly, in daily practice it is to be expected that the physician will not explicitly ask for this medication, and even if the physician asks the patient for current or previous patients, it is possible that the patient does not remember the medication. Many patients are older and take a large number of drugs, so that it is quite possible that the existing database of patient records of a physician does not provide a reliable database for whether a patient is taking drug X or not. If, however, an anonymization protocol of the anonymization software is executed and this recognizes that the currently processed patient file lacks explicit information on the taking of the drug X, then the anonymization software alone or in interoperation with the patient data management software automatically modifies the input mask 302 in such a way that the required attributes are explicitly queried, as shown here in the form of the data field 306. In addition or alternatively, the anonymization software alone or in interoperation with the patient data management software, can also generate a message, e.g. a pop-up window 308, which reminds the user to retrieve the required data or to collect them in another way (e.g. blood sampling to determine required blood values, etc.).

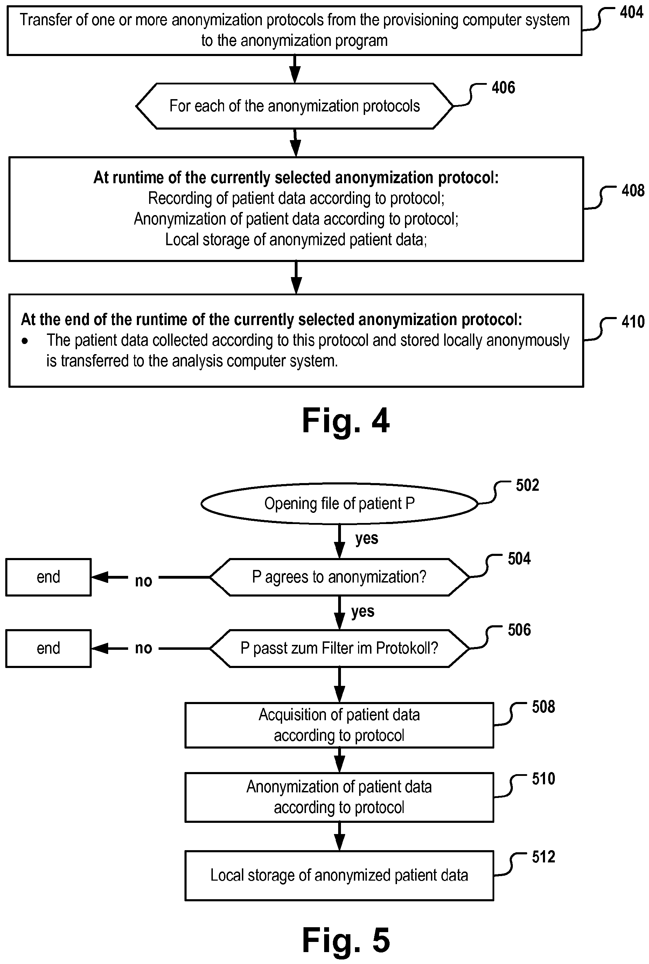

**Figure 4** shows a flowchart of a method for providing and using an anonymization protocol according to an embodiment of the invention.

The operator of the user computer system 160, e.g. a physician or a hospital manager, can contract with an operator of the control computer system, e.g. the creator of a multitude of analysis functions, for what duration and period of time anonymized patient data should be made available for which types of analysis functions and under which conditions. In the event of an agreement, one or more anonymization protocols are made available to the operator of the user computer system 160 in step 404, e.g. in the form of a download link, via which the anonymization software can download and import the one or more selected anonymization protocols from the provisioning computer system.

According to some embodiments, for each of the anonymization protocols imported into the anonymization software, which, for example, are sequentially processed in a program loop 406 on certain occasions, a part of the locally available personal files is selected in step 408 which fulfil certain criteria defined in the protocol (e.g. age, sex, medication, etc.). The occasion can be e.g. start of the anonymization software, opening of a personal file, closing of a personal file, etc. If a protocol does not specify such selection criteria, all locally available personal files are selected for further analysis by this anonymization protocol.

According to embodiments, only patient records of patients having agreed to the anonymization of their data are selected. The selected personal files are analyzed to read (capture) those attributes that are specified in the anonymization protocol as necessary for performing an analysis function. The patient data recorded according to the anonymization protocol (e.g. health status and postal code, but not X-ray images) are stored locally in anonymized form.

The anonymization software repeatedly checks all anonymization protocols it contains to see whether they have reached the end of their validity period. If the expiry date of the validity period of one of the anonymization protocols contained in the anonymization software has been reached, in step 410 all personal data records anonymized by the said anonymization protocol are collected and transmitted to the control software.

**Figure 5** shows a flowchart of a method for collecting and anonymizing personal data according to an embodiment of invention.

The method is initialized by opening 502 or creating a new personal file, e.g. in the course of a visit of the person, e.g. a patient, to the user of the user computer system, e.g. a physician. For example, a personal file can be opened by the anonymization software or by a personal data management software that is interoperable with the anonymization software.

In step 504, the physician obtains permission from the patient to transmit the patient's data anonymously. Step 506 is only executed if the patient permits the anonymization and transmission for the specific purpose of performing a particular analysis function. In this step, the anonymization program checks whether the patient fulfills the selection criteria ("filter criteria") of the analysis function at all, i.e. belongs to a certain age group, to which the analysis function should be limited. Only if this condition is also fulfilled, the anonymization software alone or in interoperation with the patient data management software in step 508 performs the acquisition of parts of the patient's data according to the protocol. "According to protocol" here means that the protocol can optionally influence the data acquisition process, e.g. by automatically modifying the fields of a GUI and/or by informing the user that data for certain attributes are still missing. If the patient refuses to consent and/or the patient does not meet the filter criteria, the patient data can still be collected or changed, but the patient data will not be anonymized or transmitted to the control software, but only stored and used locally.

In other forms, step 506 can also be performed before step 504 and step 506 can also be completely missing or missing for some of the anonymization protocols.

For the purpose of data economy, the anonymization software selectively anonymizes the attribute values of the patient file currently being processed selected according to the anonymization protocol in step 510 and saves the anonymized part of the patient file locally in step 512. The anonymization can comprise replacing concrete data values stored in a particular data field (identified e.g. as "range field" in the anonymization protocol) by a value range specified in the anonymization protocol and/or removing data values stored in a data field identified as "sensitive field" in the anonymization protocol.

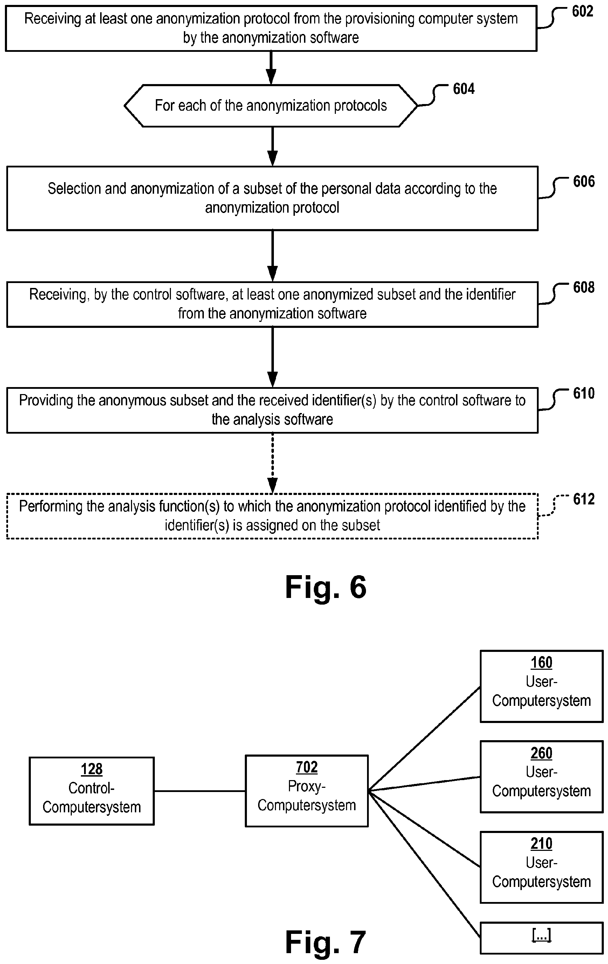

**Figure 6** shows a flowchart of a method for providing and using an anonymization protocol.

In step 602, the anonymization software receives one or more anonymization protocols from the provisioning computer system. For example, the anonymization software may be a plug-in of a patient administration program at a doctor's office and the doctor may want to participate in a particular demographic study for which the initiator of that study provides a corresponding anonymization protocol via the provisioning computer system for download. The provision can take place without any restriction in the form of a publicly accessible download link or may be access-restricted (e.g. password-protected) only to certain persons.

In some embodiments, the received protocols comprise a signature. The anonymization software performs a signature verification and integrates and locally stores selectively those protocols comprising a valid signature.

In the following steps 606-612, the anonymization protocols integrated in the anonymization software are applied to the patient data. This can be done, for example, in the form of program loops 604.

For example, when the anonymization software and/or the patient administration software is started, a program loop 604 is executed over all available anonymization protocols, regardless of whether and which patient file is currently being processed. In this embodiment or operating mode, a large number of protocols can be executed and a large number of patient files can be processed and anonymized. This operating mode is preferably executed, for example, at times when the computer on which the anonymization software is running is not used for other purposes, such as at night or on weekends.

In a different operating mode or according to different embodiments, the following steps 606-612 are performed when the physician is working in a particular patient file. In this case, the 604 program loop is only run selectively for those anonymization protocols which are stored in association with and are activated for the currently processed patient.

In step 606, a first anonymization protocol of program loop 604 is selected and executed. The execution of the anonymization protocol involves the selection and anonymization of a subset of personal data of one or more patients (for example, the personal data of a patient whose patient file is currently being processed or the personal data of several patients for whom this anonymization protocol has been activated). For example, the address information for the patient file currently being processed is only included in the anonymized data record if the address information is relevant for the analysis functions assigned to the protocol. Other possibly relevant information is at least partially anonymized by transferring concrete numerical values to numerical value ranges. Irrelevant information is omitted. The question of which attributes are relevant or irrelevant and how to make them anonymous is specified in the protocol.

In step 608, the anonymization software sends the anonymized data of one or more patients via the network to the control software and the control software receives this data. In addition to the anonymized data, an identifier of the protocol (or protocols) used for anonymization will also be transmitted or received. Depending on the mode or form of execution, the anonymized data can be transferred per patient or as a totality of anonymized data from a large number of patients. Preferably, the transmission of the patient data is separated in time from the patient's visit to the doctor, as this may allow achieving a higher degree of security for the personal data.

In step 610, the control software forwards the anonymized data received and the identifier to an analysis software that can identify the analysis functions assigned to this protocol using the protocol identifier and apply them to the anonymized data. In other embodiments, the control software can also use the protocol identifier to identify the one from a plurality of anonymization programs that implements the analysis function associated with the anonymization protocol. This can be advantageous, for example, if the control software is interoperable with many different analysis programs offered on different servers.

According to some embodiments, the method further comprises a step 612 of executing the analysis function on the anonymized data provided by the control program. For example, the analysis function can be a statistical program configured to identify correlations between zip-codes and particular illnesses.

**Figure 7** depicts a block diagram of a distributed system comprising multiple user computer systems 160, 260, 210, a control computer system 128 and a proxy computer system 702. In each of the user computer systems 160, 260, 210, personal data is collected and anonymized by an anonymization software installed in the respective user computer system. For example, user computer system 160 can be a computer in a GP's practice, computer system 260 in an oncology clinic and user computer system 210 belongs to a cardiologist. The anonymized data are encrypted by the user computer systems 160, 260, 210 with a public cryptographic key of the control computer system 128. The encrypted anonymous data is not sent directly to the control computer system 128, but exclusively via the proxy computer system 702. The proxy computer system cannot decrypt the encrypted data because the private decryption key is only accessible to the control computer system, especially the control software. Since the control computer system 128 receives the anonymized data from the proxy computer system 702, the control software cannot assign the received anonymized records to a user computer system where they were collected. The implementation variant shown in Figure 7 is particularly advantageous, since a particularly high degree of anonymization is achieved by concealing the data source.

### List of Reference Numerals

- 100: distributed computer system
- 102: database
- 104-110: personal file
- 112: database interface
- 114: anonymization software
- 116: anonymization module
- 118: filter module
- 120: one or more anonymization protocols
- 121: variety of anonymization protocols
- 122: controller interface
- 124: processor(s)
- 126: users
- 128: control computer system
- 130: analysis software
- 132-138: analysis functions
- 140: control software
- 142: data management module
- 144: database
- 146: anonymized patient data
- 148: anonymized patient data
- 150: analysis type
- 152: command
- 154: anonymized patient data
- 156: result of analysis functions
- 160: user computer system
- 200: distributed computer system
- 202: database
- 204-208: patient file
- 210: user computer system
- 212: database
- 214-218: patient file
- 260: user computer system
- 262: provisioning computer system
- 300: personal data management program
- 302: graphical user interface
- 304: dialog box for entering personal data
- 306: required data field
- 308: pop-up window
- 404-410: steps
- 502-512: steps
- 602-612: steps
- 702: proxy computer system

## Claims

1. A computer system (100) for the anonymization of personal data, comprising:
- a control computer system (128) comprising a control software (140) for providing anonymized personal data to at least one analysis software (139), the at least one analysis software comprising a plurality of different analysis functions (132-138) for analyzing personal data;
- a provisioning computer system (128, 262) comprising a plurality of anonymization protocols (121) each associated with one of said plurality of different analysis functions (132-138), each of the anonymization protocols being configured to select and anonymize personal data in a manner adapted to the one of the analysis functions associated with said analysis protocol;
- at least one user computer system (160) connected to the control computer system and the provisioning computer system via a network, the at least one user computer system comprising:
• a data store (102) in which personal data (104-110) is stored in a protected non-anonymized form;
• an anonymization software (114);
wherein the anonymization software is configured for:
- receiving at least one anonymization protocol (120) of the plurality of anonymization protocols (121) from the provisioning computer system;
for each of said at least one anonymization protocol:
- selecting and anonymizing a subset of the personal data, said selecting and anonymizing being performed in accordance with said anonymizing protocol; and
- transferring the anonymized subset and an identifier of the anonymization protocol used for anonymization to the control software;
wherein the control software is configured for:
- receiving the at least one anonymized subset and the at least one identifier from said anonymizing software; and
- providing the at least one anonymized subset and the at least one received identifier to the analysis software for performing those analysis functions to which the anonymization protocol identified by the identifier is associated, on the subset.

2. The computer system (100) according to claim 1, the control computer system further comprising:
- the analysis software (139), the analysis software being adapted to perform the one of the analysis functions identified by the identifier provided by the control software.

3. The computer system (100) according to any one of the previous claims,
- wherein the control computer system serves as the provisioning computer system; or
- wherein the control computer system and the provisioning computer systems are different computer systems.

4. The computer system (100) according to any one of the previous claims, further comprising:
- a personal data management software (300), wherein the personal data management software is configured to interoperate with the anonymization software during editing of the personal data and/or during input of new personal data by a user via a GUI (302) to compare the data currently input via the GUI and/or the input fields currently present in the GUI with the at least one anonymization protocol and to output a result of the comparison.

5. The computer system (100) according to claim 4, wherein the comparison of the data currently entered via the GUI with the anonymization protocol comprises:
- determining if and which of at least one anonymization protocol has been activated for the person whose personal data is currently being entered or edited;
- analyzing the one or more anonymization protocols activated for this person in order to determine the totality of all the attributes specified as a "necessary attribute" in all the anonymization protocols activated for this person, a "necessary attribute" being a data field of a personal file which is necessary for the execution of the analysis function assigned to the anonymization protocol;
- comparison of the determined "necessary attributes" with the entered data;
- if the entered data does not contain at least one of the necessary attributes:
• automatically outputting a warning message to the user; and/or
• automatically modifying the GUI so that the modified GUI contains input fields for at least the at least on missing necessary attributes.

6. The computer system (100) according to claim 4 or 5, wherein the comparing of the input fields currently present in the GUI with the anonymization protocols comprises:
- determining if and which of the anonymization protocols have been activated for the person whose personal data is currently being entered or edited;
- analyzing of the one or more anonymization protocols activated for this person in order to determine the totality of all the data fields specified as a "necessary data field" in all the anonymization protocols activated for this person, a "necessary data field" being a data field of a personal file used for storing an attribute that is necessary for the execution of the analysis function associated with the anonymization protocol;
- comparing the determined necessary data fields with the data fields of the GUI;
- if the GUI does not contain at least one of the necessary data fields,
• automatically outputting a warning message to the user; and/or
• automatically modifying the GUI so that the modified GUI contains input fields at least for each of the missing necessary data fields.

7. The computer system (100) according to one of the previous claims,
- the anonymization protocols each comprising a validity period, the validity period indicating a time of validity and usability of the respective protocol within the anonymization software; and
- the anonymization software being configured to automatically collect the personal data anonymized in accordance with this protocol in the form of a subset of the personal data in response to the end of the validity period and to transmit them to the control software in collected form.

8. The computer system (100) according to any one of the previous claims, wherein the anonymization software for one or more of the at least one anonymization protocol respectively comprises and continually updates a counter, wherein the one or more counters each indicate how many personal data records have already been anonymized with the anonymization protocol to which the counter is assigned, wherein the anonymization software is adapted to:
- check whether one of the counters exceeds a predefined minimum value;
- if the minimum value is exceeded, automatically collecting all personal data already anonymized by the anonymization protocol assigned to this counter and transmitting the collected anonymized personal data in the form of a batch to the control software.

9. The computer system (100) according to any one of the previous claims, wherein one or more of the anonymization protocols each include:
- a specification of one or more "sensitive data fields", wherein a "sensitive data field" is a data field of a personal file whose original content is deleted or anonymized by the anonymization protocol in the course of anonymization; and/or
- a specification of one or more "range data fields" and at least one respectively associated value range, wherein a "range data field" is a data field of a personal file whose original content is replaced in the course of anonymization by the anonymization protocol by the one of the value ranges defined in the anonymization protocol which comprises this data value; and/or
- a specification of one or more "necessary data fields", where a "necessary data field" is a data field of a personal file that is necessary to perform the analysis function associated with the anonymization protocol; and/or
- a specification of one or more "selection data fields" and at least one respective associated selection value, wherein a "selection data field" is a data field whose content determines whether or not a data field of a personal file is extracted and anonymized in the course of anonymization; and/or
- a mapping list comprising one or more synonyms mapped to a normalized term representing basically the same semantic content as the synonyms mapped to the normalized term, wherein all synonyms contained in a personal file are replaced with the normalized term to which the synonym is mapped in the protocol in the course of anonymization; and/or
- a whitelist comprising a list of allowed data values which are to be maintained in the course of anonymization;
- a blacklist comprising a list of forbidden data values which are to be deleted or replaced in the course of anonymization; and/or
- a time period indicating the granularity of an absolute-to-relative time conversion operation performed in the course of anonymization; the time period can be specified in the protocol on a per-field basis or globally for two or more different fields; and/or
- an identifier of the analysis function assigned to the anonymization protocol.

10. The computer system (100) according to one of the previous claims, the personal data consisting of a plurality of personal files, wherein the anonymization software is configured for:
- receiving a request for personal data from the control software, the request comprising an identifier of one of the anonymization protocols;
- performing said one anonymization protocol in response to receipt of said request, said one anonymization protocol comprising a specification of one or more "selection data fields" and at least one respective associated selection value, wherein performing said one anonymization protocol comprises comparing the content of said "selection data field" of all personal files with said at least one selection value, wherein said one anonymization protocol is configured to anonymize only those personal files for which said comparison provides sufficient similarity to said at least one selection value; and
- transferring the anonymized personal files as the subset of the personal data to the control software, each together with an identifier of the one anonymization protocol.

11. The computer system (100) according to any one of the previous claims, further comprising:
- a proxy computer system (702) connected via the network to the control computer system (128) and to a plurality of user computer systems (160, 260, 210) respectively comprising an instance of the anonymization software, the plurality of user computer systems including the at least one user computer system (160),
• wherein each of the plurality of user computer systems is connected to the control computer system only indirectly via the proxy computer system,
• wherein the anonymized subsets and protocol identifiers are transferred from each of the anonymization software instances to the control software via the proxy computer, and wherein the proxy computer is configured to perform the transfer such that the identity of the one of the user computers having provided any one of the anonymized subsets and protocol identifiers is hidden from the control computer; and/or
• wherein the anonymization software instantiated on each of the user computer systems is configured to encrypt the anonymized subset of the personal data such that the control software but not the proxy computer can decrypt the transferred anonymized subset of the personal data.

12. The computer system (100) according to any one of the previous claims, wherein the anonymization software is configured to perform the selection and anonymization of the subset of the personal data for the data of a plurality of persons, to collect the anonymized sub-sets and identifiers in a batch and to transfer the anonymized subsets and identifiers contained in the batch only in case the number of persons whose data is collected in the batch exceeds a predefined minimum threshold value.

13. The computer system (100) according to any one of the previous claims,
- wherein the anonymization software is configured to automatically determine the degree of anonymization achieved by the execution of the at least one anonymization protocol and to transfer the anonymized subset and identifier to the control software only in case the anonymized data guarantees a predefined minimum degree of anonymity; and/or
- wherein the control software (140) is configured to automatically determine the degree of anonymization of the transferred anonymized subset and is configured to provide the at least one anonymized subset and the at least one received identifier to the analysis software only in case the anonymized data guarantees a predefined minimum degree of anonymity.

14. The computer system (100) according to any one of the previous claims,
- wherein the provisioning computer system comprises a private cryptographic signing key;
- wherein each of the plurality of anonymization protocols comprises a signature generated with the private cryptographic signing key; and
- wherein the anonymization software comprises a public signature verification key that forms an asymmetric cryptographic key pair with the private cryptographic signing key, wherein the anonymization software is configured to verify the signature of each received protocol and for using any of the received anonymization protocols for selecting and anonymizing a subset of the personal data only in case the signature is valid.

15. The computer system (100) according to claim 14, wherein the degree of anonymization is measured as k-anonymity and/or l-diversity.

16. A computer-implemented method for anonymizing personal data, the method being performed by:
- a control computer system (128) comprising control software (140) for providing anonymized personal data to at least one analysis software (139), said at least one analysis software comprising a plurality of different analysis functions (132-138) for analyzing personal data;
- a provisioning computer system (128, 262) comprising a plurality of anonymization protocols (121) each associated with one of said plurality of different analysis functions (132-138), said anonymization protocols each configured to select and anonymize personal data in a manner adapted to said associated analysis function;
the method comprising:
- providing, by the provisioning computer system, at least one anonymization protocol (120) of the plurality of anonymization protocols (121) to an anonymization software of a user computer system (160) connected to the control computer system and the provisioning computer system via a network;
for each of said at least one anonymization protocols provided:
- receiving, by the control software of the control computer system, an anonymized subset of personal data (104-110) of one or more persons and an identifier of the one anonymization protocol used by the anonymization software for selecting and anonymizing the subset, whereby the selection and anonymization was performed in accordance with said one anonymization protocol; and
- providing (610), by the control software, the at least one anonymized subset and the at least one received identifier to the analysis software for performing the one of the analysis functions on the subset which is associated with the anonymization protocol identified by the identifier.

17. A computer-implemented method for anonymizing personal data, the method being performed by:
- at least one user computer system (160) connected to a control computer system and a provisioning computer system via a network, the at least one user computer system comprising a data store (102) in which personal data (104-110) is stored in a protected, non-anonymized form, the at least one user computer system further comprising anonymization software (114), the control computer system (128) comprising control software (140) for providing anonymized personal data to at least one analysis software (139), said at least one analysis software comprising a plurality of different analysis functions (132-138) for analyzing personal data, the provisioning computer system (128, 262) comprising a plurality of anonymization protocols (121) each associated with one of said plurality of different analysis functions (132-138), said anonymization protocols each configured to select and anonymize personal data in a manner adapted to said associated analysis function;
the method comprising:
- receiving (602), by the anonymization software, the at least one anonymization protocol (120) of the plurality of anonymization protocols (121) from the provisioning computer system;
for each of said at least one anonymization protocol:
- selecting and anonymizing (604), by the anonymization software, a subset of said personal data, said selecting and anonymizing being performed according to said at least one anonymizing protocol; and
- transmitting (606), by the anonymization software, the anonymized subset and an identifier of the anonymization protocol used for anonymization to the control software for enabling the control software to provide the at least one anonymized subset and the at least one received identifier to the analysis software for performing the one of the analysis functions on the subset which is associated with the anonymization protocol identified by the identifier.

18. A computer-readable no transitory storage medium having embedded therein a set of instructions which, when executed by one or more processors causes said processors to execute a computer-implemented method according to claim 17.

## Amended claims

### Amended claims in accordance with Rule 137(2) EPC.

1. A computer system (100) for the anonymization of personal data, comprising:
- a control computer system (128) comprising a control software (140) for providing anonymized personal data to at least one analysis software (139), the at least one analysis software comprising a plurality of different analysis functions (132-138) for analyzing personal data;
- a provisioning computer system (128, 262) comprising a plurality of anonymization protocols (121) each associated with one of said plurality of different analysis functions (132-138), each of the anonymization protocols being configured to select and anonym ize personal data in a manner adapted to the one of the analysis functions associated with said anonymization protocol;
- at least one user computer system (160) connected to the control computer system and the provisioning computer system via a network, the at least one user computer system comprising:
• a data store (102) in which personal data (104-110) is stored in a protected non-anonym ized form;
• an anonymization software (114);
wherein the anonymization software is configured for:
- receiving at least one anonymization protocol (120) of the plurality of anonymization protocols (121) from the provisioning computer system;
for each of said at least one anonymization protocol:
- selecting and anonymizing a subset of the personal data, said selecting and anonymizing being performed in accordance with said anonymizing protocol; and
- transferring the anonym ized subset and an identifier of the anonymization protocol used for anonymization to the control software;
wherein the control software is configured for:
- receiving the at least one anonym ized subset and the at least one identifier from said anonymizing software; and
- providing the at least one anonymized subset and the at least one received identifier to the analysis software for performing those analysis functions to which the anonymization protocol identified by the identifier is associated, on the subset.

2. The computer system (100) according to claim 1, the control computer system further comprising:
- the analysis software (139), the analysis software being adapted to perform the one of the analysis functions identified by the identifier provided by the control software.

3. The computer system (100) according to any one of the previous claims,
- wherein the control computer system serves as the provisioning computer system; or
- wherein the control computer system and the provisioning computer systems are different computer systems.

4. The computer system (100) according to any one of the previous claims, further comprising:
- a personal data management software (300), wherein the personal data management software is configured to interoperate with the anonymization software during editing of the personal data and/or during input of new personal data by a user via a GUI (302) to compare the data currently input via the GUI and/or the input fields currently present in the GUI with the at least one anonymization protocol and to output a result of the comparison.

5. The computer system (100) according to claim 4, wherein the comparison of the data currently entered via the GUI with the anonymization protocol comprises:
- determining if and which of at least one anonymization protocol has been activated for the person whose personal data is currently being entered or edited;
- analyzing the one or more anonymization protocols activated for this person in order to determine the totality of all the attributes specified as a "necessary attribute" in all the anonymization protocols activated for this person, a "necessary attribute" being a data field of a personal file which is necessary for the execution of the analysis function assigned to the anonymization protocol;
- comparison of the determined "necessary attributes" with the entered data;
- if the entered data does not contain at least one of the necessary attributes:
• automatically outputting a warning message to the user; and/or
• automatically modifying the GUI so that the modified GUI contains input fields for at least the at least on missing necessary attributes.

6. The computer system (100) according to claim 4 or 5, wherein the comparing of the input fields currently present in the GUI with the anonymization protocols comprises:
- determining if and which of the anonymization protocols have been activated for the person whose personal data is currently being entered or edited;
- analyzing of the one or more anonymization protocols activated for this person in order to determine the totality of all the data fields specified as a "necessary data field" in all the anonym ization protocols activated for this person, a "necessary data field" being a data field of a personal file used for storing an attribute that is necessary for the execution of the analysis function associated with the anonymization protocol;
- comparing the determined necessary data fields with the data fields of the GUI;
- if the GUI does not contain at least one of the necessary data fields,
• automatically outputting a warning message to the user; and/or
• automatically modifying the GUI so that the modified GUI contains input fields at least for each of the missing necessary data fields.

7. The computer system (100) according to one of the previous claims,
- the anonymization protocols each comprising a validity period, the validity period indicating a time of validity and usability of the respective protocol within the anonymization software; and
- the anonymization software being configured to automatically collect the personal data anonym ized in accordance with this protocol in the form of a subset of the personal data in response to the end of the validity period and to transmit them to the control software in collected form.

8. The computer system (100) according to any one of the previous claims, wherein the anonymization software for one or more of the at least one anonymization protocol respectively comprises and continually updates a counter, wherein the one or more counters each indicate how many personal data records have already been anonymized with the anonymization protocol to which the counter is assigned, wherein the anonymization software is adapted to:
- check whether one of the counters exceeds a predefined minimum value;
- if the minimum value is exceeded, automatically collecting all personal data already anonymized by the anonymization protocol assigned to this counter and transmitting the collected anonymized personal data in the form of a batch to the control software.

9. The computer system (100) according to any one of the previous claims, wherein one or more of the anonymization protocols each include:
- a specification of one or more "sensitive data fields", wherein a "sensitive data field" is a data field of a personal file whose original content is deleted or anonymized by the anonymization protocol in the course of anonymization; and/or
- a specification of one or more "range data fields" and at least one respectively associated value range, wherein a "range data field" is a data field of a personal file whose original content is replaced in the course of anonymization by the anonymization protocol by the one of the value ranges defined in the anonymization protocol which comprises this data value; and/or
- a specification of one or more "necessary data fields", where a "necessary data field" is a data field of a personal file that is necessary to perform the analysis function associated with the anonymization protocol; and/or
- a specification of one or more "selection data fields" and at least one respective associated selection value, wherein a "selection data field" is a data field whose content determines whether or not a data field of a personal file is extracted and anonymized in the course of anonymization; and/or
- a mapping list comprising one or more synonyms mapped to a normalized term representing basically the same semantic content as the synonyms mapped to the normalized term, wherein all synonyms contained in a personal file are replaced with the normalized term to which the synonym is mapped in the protocol in the course of anonymization; and/or
- a whitelist comprising a list of allowed data values which are to be maintained in the course of anonymization;
- a blacklist comprising a list of forbidden data values which are to be deleted or replaced in the course of anonymization; and/or
- a time period indicating the granularity of an absolute-to-relative time conversion operation performed in the course of anonymization; the time period can be specified in the protocol on a per-field basis or globally for two or more different fields; and/or
- an identifier of the analysis function assigned to the anonymization protocol.

10. The computer system (100) according to one of the previous claims, the personal data consisting of a plurality of personal files, wherein the anonymization software is configured for:
- receiving a request for personal data from the control software, the request comprising an identifier of one of the anonymization protocols;
- performing said one anonymization protocol in response to receipt of said request, said one anonymization protocol comprising a specification of one or more "selection data fields" and at least one respective associated selection value, wherein performing said one anonymization protocol comprises comparing the content of said "selection data field" of all personal files with said at least one selection value, wherein said one anonymization protocol is configured to anonymize only those personal files for which said comparison provides sufficient similarity to said at least one selection value; and
- transferring the anonymized personal files as the subset of the personal data to the control software, each together with an identifier of the one anonymization protocol.

11. The computer system (100) according to any one of the previous claims, further comprising:
- a proxy computer system (702) connected via the network to the control computer system (128) and to a plurality of user computer systems (160, 260, 210) respectively comprising an instance of the anonymization software, the plurality of user computer systems including the at least one user computer system (160),
• wherein each of the plurality of user computer systems is connected to the control computer system only indirectly via the proxy computer system,
• wherein the anonymized subsets and protocol identifiers are transferred from each of the anonymization software instances to the control software via the proxy computer, and wherein the proxy computer is configured to perform the transfer such that the identity of the one of the user computers having provided any one of the anonymized subsets and protocol identifiers is hidden from the control computer; and/or
• wherein the anonymization software instantiated on each of the user computer systems is configured to encrypt the anonymized subset of the personal data such that the control software but not the proxy computer can decrypt the transferred anonymized subset of the personal data.

12. The computer system (100) according to any one of the previous claims, wherein the anonymization software is configured to perform the selection and anonymization of the subset of the personal data for the data of a plurality of persons, to collect the anonymized sub-sets and identifiers in a batch and to transfer the anonymized subsets and identifiers contained in the batch only in case the number of persons whose data is collected in the batch exceeds a predefined minimum threshold value.

13. The computer system (100) according to any one of the previous claims,
- wherein the anonymization software is configured to automatically determine the degree of anonymization achieved by the execution of the at least one anonym ization protocol and to transfer the anonym ized subset and identifier to the control software only in case the anonymized data guarantees a predefined minimum degree of anonymity; and/or
- wherein the control software (140) is configured to automatically determine the degree of anonymization of the transferred anonymized subset and is configured to provide the at least one anonymized subset and the at least one received identifier to the analysis software only in case the anonymized data guarantees a predefined minimum degree of anonymity.

14. The computer system (100) according to any one of the previous claims,
- wherein the provisioning computer system comprises a private cryptographic signing key;
- wherein each of the plurality of anonymization protocols comprises a signature generated with the private cryptographic signing key; and
- wherein the anonymization software comprises a public signature verification key that forms an asymmetric cryptographic key pair with the private cryptographic signing key, wherein the anonymization software is configured to verify the signature of each received protocol and for using any of the received anonymization protocols for selecting and anonymizing a subset of the personal data only in case the signature is valid.

15. The computer system (100) according to claim 14, wherein the degree of anonymization is measured as k-anonymity and/or l-diversity.

16. A computer-implemented method for anonymizing personal data, the method being performed by:
- a control computer system (128) comprising control software (140) for providing anonymized personal data to at least one analysis software (139), said at least one analysis software comprising a plurality of different analysis functions (132-138) for analyzing personal data;
- a provisioning computer system (128, 262) comprising a plurality of anonymization protocols (121) each associated with one of said plurality of different analysis functions (132-138), said anonymization protocols each configured to select and anonymize personal data in a manner adapted to said associated analysis function;
the method comprising:
- providing, by the provisioning computer system, at least one anonymization protocol (120) of the plurality of anonymization protocols (121) to an anonymization software of a user computer system (160) connected to the control computer system and the provisioning computer system via a network;
for each of said at least one anonymization protocols provided:
- receiving, by the control software of the control computer system, an anonym ized subset of personal data (104-110) of one or more persons and an identifier of the one anonym ization protocol used by the anonymization software for selecting and anonymizing the subset, whereby the selection and anonymization was performed in accordance with said one anonymization protocol; and
- providing (610), by the control software, the at least one anonymized subset and the at least one received identifier to the analysis software for performing the one of the analysis functions which is associated with the anonymization protocol identified by the identifier on the subset.

17. A computer-implemented method for anonymizing personal data, the method being performed by:
- at least one user computer system (160) connected to a control computer system and a provisioning computer system via a network, the at least one user computer system comprising a data store (102) in which personal data (104-110) is stored in a protected, non-anonymized form, the at least one user computer system further comprising anonymization software (114), the control computer system (128) comprising control software (140) for providing anonymized personal data to at least one analysis software (139), said at least one analysis software comprising a plurality of different analysis functions (132-138) for analyzing personal data, the provisioning computer system (128, 262) comprising a plurality of anonymization protocols (121) each associated with one of said plurality of different analysis functions (132-138), said anonymization protocols each configured to select and anonymize personal data in a manner adapted to said associated analysis function;
the method comprising:
- receiving (602), by the anonymization software, the at least one anonymization protocol (120) of the plurality of anonymization protocols (121) from the provisioning computer system;
for each of said at least one anonymization protocol:
- selecting and anonymizing (604), by the anonymization software, a subset of said personal data, said selecting and anonymizing being performed according to said at least one anonymizing protocol; and
- transmitting (606), by the anonymization software, the anonymized subset and an identifier of the anonymization protocol used for anonymization to the control software for enabling the control software to provide the at least one anonymized subset and the at least one received identifier to the analysis software for performing the one of the analysis functions which is associated with the anonymization protocol identified by the identifier on the subset.

18. A computer-readable no transitory storage medium having embedded therein a set of instructions which, when executed by one or more processors causes said processors to execute a computer-implemented method according to claim 17.
